# EUROPEAN PATENT APPLICATION

(11) **EP 1 624 306 A1**
(43) Date of publication of application: **08.02.2006**
(21) Application number: 04730459.7
(22) Date of filing: 30.04.2004
(51) Int. Cl.: G01N 33/543

(54) **APPARATUS INCLUDING NANOSTRUCTURES USED FOR SEPARATION OR ANALYSIS, AND THE PREPARATION AND APPLICATION THEREOF**

(30) Priority: 30.04.2003 CN 03117787; 20.01.2004 WO PCT/CN20/04000077; 15.03.2004 WO PCT/CN20/04000203
(71) Applicant: Chengdu Kuachang Medical Industrial Limited, Chengdu, Sichuan 610041 (CN); Chengdu Kuachang Science & Technology Co., Ltd., Sichuan 610041 (CN)
(72) Inventor: ZOU, Fanglin, Chengdu, Sichuan 610000 (CN); CHEN, Chunsheng, Jinjiang Distr., Chengdu, Sichuan 610000 (CN); CHEN, Ning, Huangpu District, Shanghai (CN); WANG, Jianxia, Meishan, Sichuan 610063 (CN)
(74) Representative: Grünecker, Kinkeldey, Stockmair & Schwanhäusser Anwaltssozietät
(86) International application number: PCT/CN2004/000437
(87) International publication number: WO 2004/102196

(57) **Abstract**

This invention involves the nano-structured support used for separation or/and analysis, especially the chip substrate, ELISA plate substrate, planar chromatography strip and chromatography gel. Besides, it involves the active nano-structured support of high sensibility for separation or/and analysis, especially the analysis-chip, ELISA plate, planar chromatography reagent strip and chromatography gel. In addition, this invention also involves the nano-structured marking system for analysis. Moreover, it concerns the test kit; especially the chip kit, ELISA kit, and planar chromatography kit. What's more, this invention involes the methods of preparing all the devices mentioned above and the applications thereof, especially the chip analysis, analyses with ELISA plate, planar chromatography strip and chromatography separation.

## Description

### Technical field

This invention involves a nano-structured support, especially nano-structured substrates of chips, nano-structured substrates of ELISA plates, nano-structured strips of planar chromatography and nano-structured chromatography gel used in separation or/and analysis. This invention also involves an active nano-structured support with high sensitivity used in separation or/and analysis, especially nano-structured chips, nano-structured ELISA plates, nano-structured reagent strips of planar chromatography and nano-structured gel of chromatography. This invention further involves a marking system containing nano-structures used in analysis. Moreover, this invention involves kits, especially the chip kit, ELISA kit, and planar chromatography kit. The methods for preparation and the applications of the invention thereof, especially in the analysis with chips, with ELISA plates, with planar chromatography strips and in chromatography separation, are also provided.

### Technical Background of the invention

Supports, especially the active supports with selective reactivity has been applied extensively in many regions, especially in the fields of qualitative and/or quantitative analyses, or/and separation of targets in a sample. Affinity chromatography gel is an example of active support application whereas the antigen chip or/and antibody chip is an example of analysis application.

Most present active supports are produced by directly immobilizing active reagents (e.g. ligand) onto the surface of a support. The supports are mainly planar supports (e.g. the substrates of biochip, ELISA plate etc), granular supports (e.g. chromatography gel, especially micrometer particle chromatography gel) and membranous supports (e.g. planar chromatography strip) made from inorganic, organic materials or composites as well as their derivatives thereof. Among them, glass derivatives (such as amino-group glass slide, aldehyde-group glass slide, epoxy-group glass slide and polyamino acid-coated glass slide) are the main substrates used nowadays for biochips. The immobilized active reagent, produced by fixing the active reagent onto the substrate, is one of the key factors deciding the test sensitivity and specificity of the biochip. The available immobilization measures are mainly covalent bond immobilization, physicochemical adsorption, embedding, and cross-linking. Physicochemical adsorption includes specific adsorption and non-specific adsorption. Affinity adsorption is an example of the former, whereas ionic adsorption is an example for the latter. Antigen-antibody reaction, nucleotide coupling reaction, and oleophilic reaction all belong to affinity adsorption. The reaction kinetic condition for active reagents is to be optimized and detailed, which is manifested as improved sensitivity or/and specificity; or/and to shortened reaction time.

How to improve the immobilized active reagent through modifying the surface of a support has long been a major problem in analysis or/and separation research and development, and has attracted thousands of laboratories worldwide. Though many researchers are working on the modification of the surface of support by using nano-particles or colloid, they either fail to take the sensitivity as the primary goal (e.g. WO 0183825), or fail to take the immobilized active reagents as the main target (e.g. USA Patent Application No.20030207296), or obtain the support at an extremely high cost (e.g. the oriented and discontinuous hypomicrometer whisker structure made by USA 3M Comp, US Patent Application No: 5709943 and Chiese Patent Application No: 96013700.9). In the document of WO 0183825, colloid, especially the organic particles at size of 100-500nm is used as the ligand support, so as to decrease the workload in chip manufacturing, e.g. the processing procedures are so simplified as to produce chips with uniform probe spots. In the USA Patent Application No: 20030207296, the emphasis is laid on taking the nano-particle itself as the ligand support in the nucleic acid test. However the research on the modified nano-particle substrate is inadequate.

Another key factor for sensitivity and test time is the marking system. The prevalent marking system today is a molecular dispersion marking system whose sensitivity is yet to be improved, in which hundreds of labs are engaged. Some application of nano-particles has been introduced into the present marking systems in order to increase test sensitivity (such as WO 00/72018 A1, US Patent Application No: 20030211488, Chinese Patent Application No: 02133538.9, 02137418.X, 01133527.0, whose research thereof uses colloidal gold as the label, supplemented with silver enhancement system), but these particles are either labeling materials themselves (e.g. China Patent Applications No: 02141718.0, 01109551.2, whose research thereof uses fluorescent rare earth compound nano-particles as label), or reinforcing agents (e.g. US Patent Application No: 20030232388, 20030166207, 20020142480, 20030211488, whose research thereof uses metals with raman enhancement effect). There are some patents wherein some nanometer crystal particles are employed as identifying codes of nano-particle supports, which connect molecules with varied activities (e.g. US. US Patent Application, No: 6544732). In addition, active supports are used extensively in separating devices such as chromatography devices, e.g., the functional matrix of affinity chromatography. Although the particle employed by the functional matrix of chromatography has larger surface region than baseplate, it leaves much to be desired in terms of chromatography time, and rate etc.

### Invention content

The main objective of this invention is to increase the reaction efficiency of active supports or/and labels in analysis and separation at relatively lower cost, thereby increasing the test sensitivity or/and shortening the test time or/and providing more supports with adequate sensitivity. The objective is attained through the development of supports, active supports and marking systems. Based on the nature of the research, i.e. that study of the support and active support, improvement of separation media as well as their efficiency becomes the additional objective of the invention.

The primary facet of the invention is to provide, for the purpose of separation or/and analysis, a nano-structured support that comprises nano-structured regions, wherein there are conventional supports and nano-structures fixed on the former. The nano-structures comprise nano-structure units which consist of nano-convexes in non-orientational arrangement pattern, wherein the said nano-convexes have a height of more than 3nm and present at least one dimensional size of 1-500nm, optimally 1-100nm at the half-height, and the nano-convexes are distributed in the nano-structured region with a distribution density of more than 1 nano-convex /µm² , optimally more than 10 nano-convex /µm² .

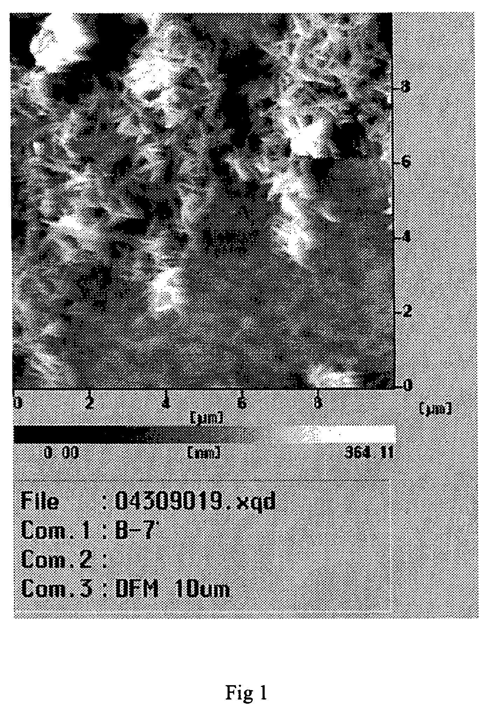

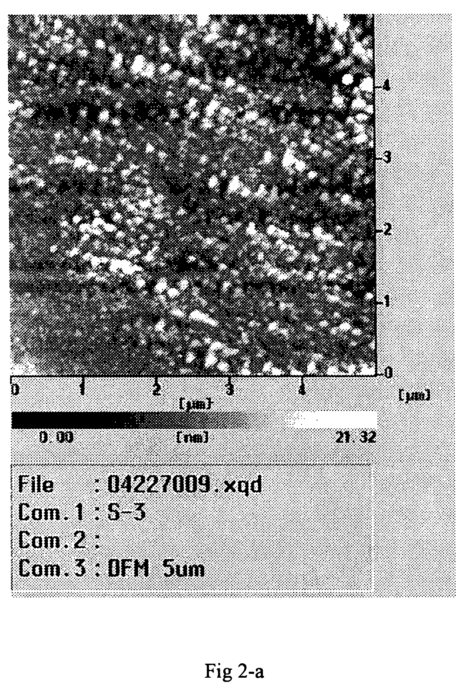

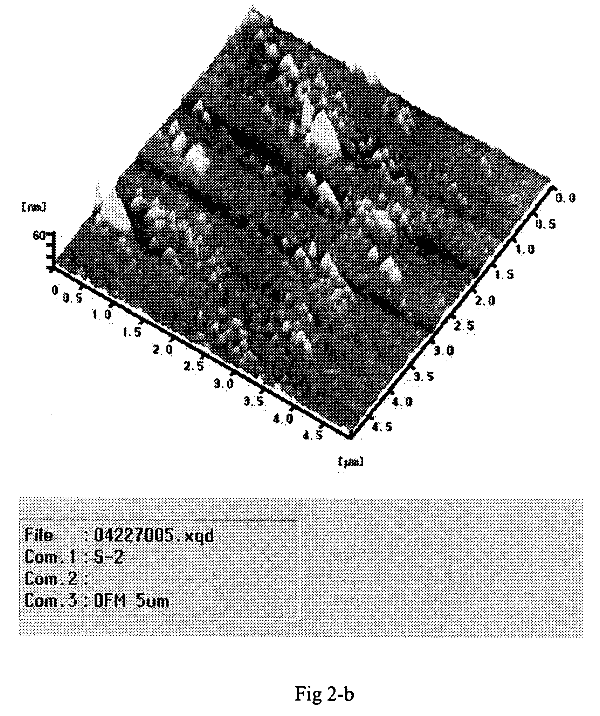

The term "nano-convex" of the invention refers to a protruding object with at least one-dimensional size of 1-1000nm, and it presents only one head, such as a nano-particle fixed on a support or other structures; the term "nano-structure unit" refers to a structured unit with at least one-dimensional size of 1-1000nm, such as a nano-particle; the term "nano-structure" refers to a structure composed of nano-structures whose height is less than 1000nm, such as a nano-structure unit or nano-convex object in the shape of a tree branch, hillock, net or other geometric figures, as well as refers to the correlation between nano-structure units or nano-convex objects (e.g. the branch-shaped object may be upwards or parallel to the surface etc; Diagram 1 will illustrates these nano-convexes or the branch-shaped objects containing nano-convexes;

The term "nano-structured region" refers to an region of the nano-structured support, where the specific characteristics of the said invention present (e.g. the distribution and distribution density of nano-structure units etc), including the said nano-structure and the optional surface of conventional supports that have no fixed nano-structure thereon; the term "non-nano-structured region " refers to an region on nano-structured support beyond the nano-structured region. Non-nano-stuctured region may contain nano-stuctures but without the features of nano-structured region. The invention of nano-structured support includes nano-structured region and non-nano-strctured region. The separated or/and assayed targets include one or more groups of the following materials: polypeptides, pharmaceutics interacted with polypeptides, nucleic acids, pharmaceutics interacted with polypeptides.

According to the primary facet of the invention, namely the nano-structured support, the nano-structure coverage rate of the said nano-structured region is over 20%, optimally more than 30%. In the invention, the term "nano-structure coverage rate" refers to the ratio between the surface region of the conventional support covered by the nano-structure and the total surface region of the conventional support: the nano-structure coverage rate = the surface region of the conventional support covered by nano-stuctures / the total surface region of the conventional support.

According to the primary facet of the invention, namely the nano-structured support, the surface-increasing rate of the nano-structured region is more than 200%, preferably more than 300%, optimally more than 400%.In the invention, the term "the surface-increasing rate" refers to the ratio of the surface region between the nano-structured region and the conventional support surface region therein: the increasing rate of surface region = (the nano-structured surface region / the conventional support surface region therein) × 100%. The rate can be measured directly or indirectly.

According to the primary facet of the invention, namely the nano-structured support, the adsorbability-increasing rate of the nano-structured region is more than 115%, preferably more than 130%, optimally more than 150%. In the invention, the term "adsorbability increasing rate of the nano-structured region " refers to the ration of the adsorbability between the nano-structured region and that of the conventional support therein: the adsorbability increasing rate = (the adsorbability of the nano-structured region / the adsorbability of the conventional support therein) X 100%. The selection of the adsorbed material in measurement goes in accordance with the established method.

According to the primary facet of the invention, namely the nano-structured support, the adsorption attenuating rate of the nano-structured region is less than 90%, optimally less than 80%. In the invention, the term "adsorption attenuating rate of the nano-structured region " refers to the ratio between the adsorption attenuating velocity of the nano-structured region and that of nonnano-structured region on the same support as the target concentration decreases: the adsorption attenuating rate = (the adsorption attenuating velocity of the nano-structured region with the decrease of targets concentration / the adsorption attenuating velocity of the conventional support therein with the decrease of targets concentration) × 100%.

The nano-structured support in this invention is of the specific features of nano-structure (e.g. the nano-structured region, distribution and distribution density of nano-structure units therein, coverage rate etc.) as well as properties of nanometer (e.g. the surface-increasing rate, absorbability increasing rate, adsorption attenuating rate, etc.). The nanometer characteristics of the said nano-structured support may or may not be correlated with the said nano-structure.

The nano-structured support in this invention is of specific features of nano-structure and property of nanometer, which differs from the "nano-particle-coated support". As a matter of fact it is easy for the conventional support to have nano-particles thereon, as a result of defects in manufacturing, or result of non-technical dust adsorption, etc, but they are not the nano-structured supports mentioned by this invention. Though supports can be technically coated with nano-particles, the nano-particle-coated supports attained thereby, in most cases, still do not possess the features of the said invented nano-structured support, especially the features of nano-structured region, (e.g. in terms of the surface increasing rate, adsorbability increasing rate, adsorption attenuating rate etc.). With no distinction from the conventional support in nature, they are not at all the nano-structured supports of the invention.

The nano-structured supports of the invention can be obtained through various approaches. However, the said preparation of this invention is the optimized method of obtaining the nano-structured support, which places emphasis on an optimized concentration of nano-particle. In an implementation example of the invention, the nano-particle-coated supports, prepared not under the condition of optimized concentration, do not have the features of the said nano-structured supports, so they are not the nano-structured supports of this invention.

In the nano-structured support considered as the primary facet of the invention, the nano-structure unit is made of nano-particles with a diameter of 1-500nm, preferably 1-100nm, and optimally 1-50nm. The binding between the said nano-particle and support, or/and among the nano-particles can be established through one or more ways, such as: covalent bond linking, non-specific physicochemical adsorption, antigen-antibody adsorption, affinity adsorption, nucleotide coupling conjugation, and heat polymerization below the softening temperature, etc. Optimally, the said binding works through linking agents on the surfaces of the support or/and nano-particles. The linking agents include surface group or/and coating organic.

In the nano-structured support considered as the primary facet of the invention, the mentioned nano-particle includes inorganic, organic nano-particles, or/and their derivatives thereof The mentioned inorganic nano-particle includes non-magnetic inorganic nano-particles and magnetic inorganic nano-particles.

The said non-magnetic inorganic nano-particle includes non-magnetic metal nano-particles and non-magnetic non-metal nano-particles.

The said derivatives of the inorganic nano-particle or/and organic nano-particle include derivatives bound with the surface group or/and coated with organisms. The said non-metal nano-particles include organic nano-particles such as those of plastic, polysaccharides emulsion and resin. The said inorganic non-magnetic non-metallic nano-particles comprise oxide nano-particles such as those of silica oxide, titanium and aluminium. The non-magnetic metal nano-particles include gold, vanadium and lead particles. Metal particles, like gold nano-particle, have been used as rapid reagent strips, but they are still used as molecular labeling particles rather than the support used in this invention.

In the nano-structured support considered as the primary facet of the invention, the conventional support includes supports the size over 1000nm, made from one or more groups of following materials or the derivatives whereof: glass, silica, silica gel, ceramics, metallic oxide, metals, polymer material as well as their compounds, wherein the said derivatives includes those bound with surface groups or/and those coated with organisms. The said supports include the planar supports (e.g. the substrates of biochips, ELISA plates etc), granular supports (e.g. the chromatography gel, especially micrometer particle chromatography gel) and membranous supports (e.g. plane chromatography strip).

In the nano-structured support of the first aspect of the invention, the said surface group includes one or more of the following groups: amino-groups, aldehyde-groups, epoxy-groups, amino diazane, diethylaminoethyl, diethyl- (2-hydroxypropyl) aminoethyl, carboxymethyl, sulfopropyl, mercaptoethylpyridine, siloxanyl, thiol groups and alkyl groups;

The coating organic compounds include one or more of the following organisms: surface active agents including polyvinylpyrrolidone and Tween, polyelectrolyte including polyamino acid, oleophilic organisms including polysiloxane, ion exchange polymers including dextran derivative, agarose derivatives, cellulose derivatives, polyacrylamide, and affinity materials including heparin natrium, antigens and antibodies.

The surface groups could also be long-armed derivative group R―(CH2)x―, wherein R stands for the derivative group, x is no less than 2, preferably larger than 4, optimally larger than 6. In this invention, the active organic groups combinable with the surface of nano-particle cover a wide range, particularly because of the preparation of coated derivatives. For instance, in implementation examples of the invention, when there is an alkyl-group on the nano-particle derivatives such as super-hydrophobic silica (CS7), the coated derivative is covered by coating organic groups, such as polyamino acid with amino-groups, amino diazane with amino and amino diazanyl-groups, DEAE-Dextran with diethylaminoethyl, etc.

On the other hand, surface-active agents like polyvinylpyrrolidine, polyelectrolyte like polyamino acid, ion exchange polymers like DEAE-Dextran, affinity materials like Protein A etc. are applied in this invention for the preparation of ligand / nano-particles / substrate complexes and ligand / nano-particles / molecular labeling complexes. In fact, the importance of particle derivatives lies in the coated derivatives. The coated derivatives in the invention can be monocoated or multicoated. For example, silica particles are coated with dispersants or/and dispersing stabilizers (simplex coating), then with PVP (duplex coating), and even further with Protein A (triplex coating). Hence, available coating organic compounds are numerous. The surface groups or/and coating organic compounds can be fixed on nano-particlse or/and conventional supports.

In the nano-structured support considered as the primary facet of the invention, the nano-structured supports include: complex of nano-particle / substrate, complex of nano-particle / micro-particle or complex of nano-particle / micro-particle / substrate. What is to be emphasized is that the invented nano-particle / substrate complex, nano-particle /micrometer particle complex or nano-particle / micrometer particle / substrate complex is used as those of the invented nano-structured support.

The nano-structured support considered as the primary facet of the invention can be used as the nano-structured substrate of the analysis-chip; nano-structured microplate for the enzyme-labeling plate; nano-structured substrate for planar chromatography; nano-structured matrix for separation; nano-structured matrix for separation including nano-structured chromatography gel.

The secondary facet of this invention is to provide a method preparing the nano-structured support specified by the primary facet of this invention, wherein the said nano-particle suspension is made to contact and have an immobilizing reaction with the said conventional support, in which the suspension presents a nano-particle concentration of 1/500 to 1/150000 in weight/volume concentration (g/ml) or 0.12-37.4nM in mole concentration. To have a definite nano-particle concentration is one of the key factors in the method preparing the invented nano-structured support. The nano-structured support prepared accordingly can be used as the substrate or path of analysis-chips, the substrate of ELISA plates, the substrate of planar chromatography reagent strips and the support of chromatography gel etc.

In the method considered the secondary facet of this invention, the nano-particle presents a weight/volume concentration of 1/5000 to 1/150000 (g/ml), optimally 1/20000 to 1/60000 or mole concentration of 0.12-3.74nM, optimally 0.31-0.93nM.

The method considered the secondary facet of this invention also includes one or more of the following functionalizational processes to help the bonding with other related materials:
(a) First functionalize the nano-particles or/and conventional supports, and then proceed with the said contact and immobilization;
(b) First execute the said the contact and immobilization of the nano-particles or/and conventional supports, and then proceed with the functionalization.
(c) First functionalize the nano-particles or/and conventional supports, next proceed with the said contact and immobilization and finally re-functionalize the nano-particles or/and conventional supports;

The said functionalization thereof includes the employment of the surface groups or/and coating organic compounds.

The method considered the secondary facet of this invention also involves the chemical cross-linking process for the products of the immobilizing reaction. Crosslinking agents usable in the invention include coupling agents, such as epoxy chloropropane, urotropine, etc.

The method considered the secondary facet of this invention also involves heat treatment of the products of the immobilizing reaction. The heat treatment includes the heating and the subsequent cooling at over 30°C, preferably over 37°C, optimally over 40°C, but below the sintering point of the said support. In existing techniques, one of the measures to improve the stability of nano-particle-coated substrates lies in the heat treatment of the support sintering point. However, heating at the sintering point tends to bring about deformation of the support, causing difficulties in application. Surprisingly enough, though the heat treatment in this invention is conducted at the temperature a dozen or even a hundred degrees below the sintering point, it is still able to create a strong bonding between the support and nano-particles. This invention is not intended to discuss the theory beyond the assumption that nano-particles may possess heat characteristics different from other conventional materials. The heat treatment method of this invention will cause no deformation of supports.

The third facet of this invention is to provide an active nano-structured support for separating or/and analytical reactors, which comprises active nano-structured regions, wherein the active nano-structured regions comprise the conventional support and immobilized active nano-structured thereon. The said active nano-structure comprises non-orientationaly arrayed nano-structure units and immobilized active reagents thereon, wherein the nano-structure units include nano-convex objects with a convex height of more than 3nm, and at least with one-dimensional size of 1-500nm, optimally 1-100nm at the half-height. And the distribution density of the nano-convex objects in the nano-structured region is > 1/µm², optimally >10/µm².

In this invention, the term "active region" refers to the region on which the said active reagent is fixed. It can comprise the complete support or one or several mutually independent regions of the support (e.g. microarray chip); the term "non-active region" refers to regions other than the active region; the term "active nano-structured region" refers to the region inside the active region, which not only contains the above mentioned active reagent, but also possesses specific features (e.g. the distribution of nano-structure units and distribution density etc) of this invention. It includes the said nano-structure, active reagent fixed on the nano-structure and optionally existent surface of the conventional support without the immobilized nano-strucuture mentioned-above (in Picture 2, these nano-convex objects or the branch-shaped object with nano-convex object could be seen). The active nano-structured region may comprise the complete support or just one or several mutually independent regions of the support (e.g. nano-structured chip).

In this invention, the term "nano-structured chip" refers to a chip that contains at least one said active nano-structured region (e.g. one spot in probe microarray). Such a chip may have several reactors and one reactor may contain several spots with active reagents (probe spots). If one spot is the active nano-structured region defined by this invention, the whole chip will be regarded as the active nano-structured support or nano-structured chip of this invention.

The term "non-active nano-structured region" refers to regions other than the active nano-structured region inside the active region. It may contain nano-structures but does not have the features of active nano-structured region. The terms "nano-convex object", "nano-structured unit", "nano-structure", "nano-structured region ", "non-nano-structured region" have been defined in the above paragraphs. Active nano-structured supports include active regions and optionally existent non-active regions. The active region includes active nano-structured region and optionally existent non-active nano-structured region. In the invention, the active reagents are those endowed with the said active nano-structured support with reactivity (e.g. the reactivity with target), such as ion exchangers, pharmaceutics, polypeptides, polysaccharides, vitamins, antibiotics, functional organics, antigens, and viruses, cells or their compositions. The active nano-structured supports in this invention do not contain labeling material (e.g. the biochip without fluorescein), for it is used in reactors only.

One thing to be specially emphasized is that it is the specific features of nano-structure and the property of nanometer that make the invented active nano-structured support different from active support with nano-particle in general. Though the active support in some literatures (such as No.20030207296 of USA Patent Application) is the one with the active support and nano-particles thereon, surprisingly we have found from a great many experiments that active supports with different features (e.g. varied appearances) present absolutely different activities (e.g. test sensitivity). The active nano-structured support in this invention has specific features, thereby possessing definite high activity (e.g. test sensitivity). In addition, the Self-assemble metal colloid monolayers claimed by US 6025202 of USA Patent differ radically from the invented nano-structured support in the technical design. It does not have the features of active nano-structured support of this invention. Actually, active nano-particles affixed on the surface of supports will not necessarily form the active nano-structured regions described in the invention.

In the active nano-structured support considered the third facet of the invention, the coverage rate of the nano-structure in the nano-structured region is more than 30%, optimally more than 75%. The term "coverage rate of nano-structure in the active nano-structured region" of the invention refers to the ratio between the surface region of the conventional support coated with the said nano-structure and the total surface region of the conventional support in the nano-structured region: the coverage rate of nano-structured = the nano-stucture coated surface region of the conventional support / the total surface region of the conventional support.

In the active nano-structured support considered the third facet of the invention, the surface increasing rate of the active nano-structured region is more than 200%, preferably more than 400%, optimally more than 600%. The term "surface increasing rate of the active nano-structured region" of the invention refers to the ratio of surface region between the active nano-structured region and the conventional support therein: the surface increasing rate = the surface region of active nano-structured region / the surface region of the conventional support therein) × 100%. The surface-increasing rate can be measured directly or indirectly. In one of the implementation of the invention, the surface increasing rate of an analysis-chip is measured indirectly by coating the active nano-particle and active reagent respectively on the whole substrate. The surface-increasing rate in the invented chip is up to 8.0 or more.

In the nano-structured support considered the third facet of the invention, the sensitivity increasing rate of active nano-structured region is more than 120%, preferably more than 150%, optimally more than 200%, or/and the adsorbability increasing rate is more than 115%, preferably more than 130%, optimally more than 150%. The term "sensitivity increasing rate of the active nano-structured region" of this invention refers to the ratio of analytical sensitivities between the active nano-structured region and the nonnano-structured active support which possesses the same conventional support and active reagent: sensitivity increasing rate = (the sensitivity of active nano-structured region / the sensitivity of nonnano-structured active support) × 100%. The term " adsorbability increasing rate of the active nano-structured region" refers to the ratio of the adsorbability between active nano-structured region and the nonnano-structured active support which possesses the same conventional support and active reagent: adsorbability increasing rate = (adsorbability of active nano-structured region / adsorbability of nonnano-structured active support) X 100%. The term "nonnano-structured active support" refers to active supports without the active nano-structured region of this invention, such as the chip prepared by loading HCV antigens onto epoxy-group glass slides, whereas an example of the active nano-structured support is the said chip of the invention prepared by looading the suspension of HCV antigen-coated silica nano-particles onto epoxy-group glass slide). In the measurement of adsorbability, the selection of the adsorbed material depends on the immobilized active reagent. The sensitivity-increasing rate of the invented chip is up to 300% or more.

In the nano-structured support considered the third facet of the invention, the attenuating rate of signal identification in the said active nano-structured region is less than 90%, optimally less than 70%, whereas the adsorption attenuating rate is less than 90%, optimally less than 80%. In the invention, the term "attenuating rate of signal identification in the active nano-structured region" refers to the ratio of attenuating velocity as the target concentration decreases between the active nano-structured region and nonnano-structured active support which possesses the same conventional support and active reagent: attenuating rate of signal identification in the active nano-structured region = (attenuating velocity of signal identification in the active nano-structured region with the decrease of targets concentration / the attenuating velocity of signal identification in the nonnano-structured active support with the decrease of targets concentration) x 100%.

The term "adsorption attenuating rate of the active nano-structured region" refers to the ratio of adsorption attenuating velocity between the nano-structured region and nonnano-structured active support which possesses the same conventional support and active reagent as the target concentration decreases: adsorption attenuating rate of the active nano-structured region = (the adsorption attenuating velocity of the active nano-structured region with the decrease of targets concentration / the adsorption attenuating velocity of the nonnano-structured active support with the decrease of targets concentration) × 100%.

The active nano-structured support of this invention differs from the bioactive support described in PCT International Patent Application PCT/US96/04485 (and CN96193700.9) in its technical design. The active nano-structure of the latter is composed of oriented nanometer whiskers. Its optimal preparation also differs from that of this invention. Consequently, it is of high cost, and not able to fix active nano-particle array, as the chip of this invention does, onto places of the support as required.

The active nano-structured support of this invention differ also from other well-known active nano-structured supports, for it possesses specific features of nano-structures (e.g. the distribution and distribution density of the active nano-structured regions and nano-structure units therein, the coverage rate of nano-structure etc.) as well as properties of nanometer (the surface increasing rate, adsorbability increasing rate, adsorption attenuating rate, sensitivity increasing rate, attenuation rate of signal identification, etc.).

The properties of nanometer in the said active nano-structured support may be related to the nano-structures described above, or the nano-structures not mentioned yet. The active nano-structured support of the invention, with the specific features of nano-structure and properties of nanometer, is as different from "nano-particle-coated supports containing active reagents" as from "nano-particle-coated supports" in a general sense.

The active nano-structured support of the invention can be obtained via various approaches, but the said method of its preparation is the optimal one. One thing about the preparing method needs to be emphasized: an optimal concentration of active nano-particle is one of the keys for obtaining the active nano-structured supports of the invention. In an implementation example of the invention, the nano-particle-coated active supports prepared not under the said optimal concentration condition, do not have the features of the active nano-structured support, so they are not the invented active nano-structured supports. We will discuss and explore the differences, like that in appearance in an implementation example. With the help of electronic probe microscopes (DFM) and electronic scanning microscopes, the observed convex object whose size is far bigger than that of nanometer may be nano-particle aggregates. Furthermore, large-sized nano-particle aggregates may lose or partially lose features of nano-structures at least. For example, we found in the implementation examples that the active support is generated when the concentration of active silica is over 1%, wherein this kind of convex object (non-active nano-structured region) has a much larger proportion in the active region, therefore, presenting a much lower test sensitivity.

In conclusion, active supports containing active nano-particles generated without strict structural design are different from the active nano-structured support of this invention. The active support with the features described above is the active nano-structured support of the invention. Additionally, the invented active nano-structured support includes simplex active nano-structured support and multiplex active nano-structured support, wherein the said simplex active nano-structured support is made by fixing the active nano-particle containing only one type of active reagent onto an active support; whereas, the multiplex active nano-structured support is made by fixzing the active nano-particle containing more than two types of active reagents onto an active support. In No.20030207296 USA Patent Application, there is only simplex active nano-structured support. A biochip and a planar chromatography reagent strip are the examples of device containing multiplex active nano-structured support; whereas an ELISA plate is the example of device containing simplex active nano-structured support.

In the active nano-structured support considered the third facet of the invention, the separated or/and analyzed targets include polypeptides or/and the pharmaceutics interacted with polypeptides. It is well known that there are many active reagents that can react with target polypeptides, ion exchangers, pharmaceutics, polypeptides, polysaccharides, vitamins, antibiotics, functional organics, antigens, and viruses, cells or their compositions as some of the examples. In this type of active nano-structured supports, the active nano-structure could be bound to the support not through nucleotide coupling conjugation, but by one or several approaches mentioned below: covalent bond linking, nonspecific physicochemical adsorption, antigen-antibody adsorption, and affinity adsorption. And the bonding is completed with the help of the linking agent on the surface of supports or/and nano-particles. The linking agents include surface groups, coating organic compounds or/and the active reagent. In No.20030207296 USA Patent Application, the bonding of nano-particles and substrates must depend on nucleic acid coupling effect, so it is unsuitable for detecting and separating other materials, except nucleic acids like polypeptides.

In the active nano-structured support considered the third facet of the invention, the said separated or/and analyzed targets include nucleic acids or/and drugs interacted with nucleic acids. The nucleic acid nano-particle-coated substrate of the invention differs from that claimed by No20030207296 USA patent application, because the former is of the specific feature of nano-structures and properties of nanometer.
The active nano-structured support considered the third facet of the invention could be the complex of active nano-particle and support. The active nano-particle comprises nano-particle and one or several types of active reagents immobilized thereon or optionally existent linking agent. The size of the nano-particle is 1-500nm, 1-100nm preferably, and 1-50nm optimally. The support is the conventional support or the nano-structured support according to any claim from 1 to 14. The active nano-particle/substrate complex can be obtained by many approaches. However, this invented method of preparing active nano-particle/substrate complex is the optimal one.

In the active nano-structured support considered the third facet of the invention, the nano-particle includes the nano-particle considered the primary facet of the invention. The inorganic non-magnetic non-metal nano-particle and the derivatives thereof are the optimal selection. Moreover, its structural features are different from those described in USA patent 6025202 and PCT international patent application PCT/US96/004485 (and CN96193700.9). Besides, its optimal design also includes the active nano-structured support without metal surfaces and metal nano-particles, whereas in USA patent 6025202, the surface of nano-struture is coated with metal, and in PCT international patent application PCT/US96/004485 (and CN96193700.9) colloid gold is employed (colloid gold is fixed on organic membrane, while the organic membrane is fixed on substrate). Therefore the manufacture of the active nano-structured support of this invention is simpler and the cost is lower.

The active nano-structured support considered the third facet of the invention includes one of the following: active nano-particle/substrate complex, active nano-particle / micrometer particle complex, active nano-particle / nano-structured substrate complex, active nano-particle / nano-structured micrometer particle complex as well as their combinations.

The active nano-structured support considered the third facet of the invention could be the compound of active reagent and the nano-structured support considered the primary facet of the invention.

The active nano-structured support considered the third facet of the invention includes one of following selected groups: active reagent/nano-structured substrate compounds, active reagent/nano-structured micrometer particle complex, as well as their combinations. The active nano-particle/ substrate complex can be obtained by various approaches. However, this invented method of preparing active nano-particle/substrate complex is the optimal preparing method in the invention.

In the active nano-structured support considered the third facet of the invention, there are simplex or multiplex ligands between one or more types of the nano-particles and the support, or/and simplex or multiplex nano-particles between one or more types of the ligands and the support, or/and at least simplex or multiplex ligands between simplex nano-particles and other simplex nano-particles. The active nano-structured support with multiplex ligands between one or several types of nano-particles and support is prepared as such: simplex ligand① is used to coat the support to create ligand①-coated support, simplex ligand ② is used to the coat nano-particle to create the complex of ligand ② nano-particle(coupling reaction may occur between ligand ① and ligand ②), and then the compound of ligand ② / nano-particle is used to coat or is loaded onto ligand① -coated support to create the complex of ligand②-nano-particle-ligand ②-ligand①-support. When the layer of ligand is more than 2, similar method will be adopted. There are many types of active nano-structured supports with multiplex ligands between the simplex nano-particle and other simplex nano-particle, e.g. ligand③-nano-particle-ligand③-ligand ②-nano-particle-ligand②-ligand①-support or ligand②-nano-particle-ligand②-ligand① -nano-particle-ligand①-support, etc. There are active nano-structured supports containing multiplex nano-particle between one or several types of ligands, and the support can be prepared as such: first combine one or more types of the nano-particles with above mentioned ligands to form several types of active nano-particles (e.g. ligand ② -nano-particle-ligand, ligand③-nano-particle-ligand②, ligand①-nano-particle-ligand①, etc.), and then these active nano-particles are bound to the supports successively or simultaneously, yielding active nano-structured supports of ligand② - nano-particle - ligand② - ligand①- nano-particle - ligand① - support, ligand③ - nano-particle - ligand ② - ligand① - nano-particle - ligand① - support, etc.

The active nano-structured support considered the third facet of the invention can be used as the nano-structured analysis-chip. In addition, it can be used as the nano-structured ELISA plate, nano-structured planar chromatography reagent strip, nano-stuctured active matrix for separation including active nano-structured chromatography gel.

The fourth facet of this invention is to provide a method of preparing the active nano-structured support considered the secondary facet of this invention, which involves bringing the suspension of active nano-particle into contact with the conventional support as well as the immobilization reaction. In the immobilization reaction, the nano-particle in the suspension presents weight/volume concentration of 1/500 to 1/150000(g/ml), or mole concentration of 0.12-37.4nM; the contact refers to either point contact with a contact diameter less than 0.5mm, or surface contact with contact diameter more than 0.5mm. The following is an example of forming the invented complex of active nano-particles and the support: nano-particle suspension is mixed and reacted with ligand solution to generate active nano-particles, and then the mixed solution is spotted onto the substrate of baseplates on chip to have binding reaction etc.

The support in the invention can also be prepared through methods shared with other structures, e.g. the substrate with multi-wells etc. The said active nano-particle could be prepared by mixing nano-particle and ligands with other materials, e.g. nano-particle suspension containing colorants or/and adhesives, ligand solution containing stabilizers, etc. The said suspension of active nano-particles includes the mixture (e.g. unpurified products after the mixing reaction between nano-particle and ligand) and purified substance (e.g. the purified products free of ligand by centrifugal separation after the mixing reaction between nano-particle and ligand), and on one type of nano-particles in the purified substance, one or several types of ligands are immobilized. One thing that is to be emphasized particularly is: when nano-particles are either under or over diluted, the said complex prepared from the nano-particles will present no increase of sensitivity.

In the method considered the fourth facet of the invention, the nano-particle weight/volume concentration (g/ml) is 1/5000 to 1/150000, or nano-particle mole concentration is 0.12-3.74nM. Optimally, the nano-particle weight/volume concentration (g/ml) is 1/20000 to 1/60000, or 0.31-0.93nM for nano-particle mole concentration.

The method considered the fourth facet of the invention also includes chemical cross-linking process for the reactant after the immobilization reaction. Moreover, it includes heat treatment for the reactant after the immobilization reaction. The heat treatment includes the heating at over 30°C, preferably over 37°C, optimally over 40°C, but below the sintering point of support, with or without the protection of active reagent protectant and the subsequent cooling. The said active reagent includes one or more of stabilizers: amino acid, protein and sugar.

The fifth facet of this invention is to provide an analytical or/separating device, which comprises the nano-structured support considered the primary facet of the invention or/and active nano-structured support considered the third facet of the invention, including the devices used for analyses of polypeptides, nucleic acids or/and drugs. The invented active nano-structured support can be used as analysis-chip, ELISA plate, planar chromatography reagent strip, chromatography gel and their assembled devices.

The devices considered the fifth facet of the invention include one of the analysis-chips of the following: microarray chip, microarray-channel chip and channel chip, wherein the said channels include the nano-structured channel with the nano-structured support or/and the active nano-structured channels with active nano-structured support. The invented analysis-chip is the core of this invention.

One of the invented analysis-chips is the chip with channels. At present, the microchannel or microflowing path is the most widely studied subject among all the reactor channel structures and reactor separating structures. The size of microchannel in general is less than 0.10mm in width, and less than 0.025mm in depth, therefore a concave channel. The chip with microchannels has been called microchannelled chip; that is a miniature of a comprehensive analytic system which employs microchannels as the network to connect micropump, microvalve, microvessel, microelectrode, microanalysis units etc. so that it can combine sampling, pre-processing, liquid-delivering and other test functions into one.

Generally, in every reactor of microchanneled chip, only one type of ligand is immobilized, with only one target in the sample detected at one test. An example for microchanneled chip is the analysis-chip of Caliper Technologies Inc. (www.caliper.com). The strength of microchannel biochip is its high sensitivity and fast speed. The weakness is: 1) in the process of manufacturing, the microchannel should be etched beforehand. Then probe is deposited thereon. After that, the microchannel is enclosed. Owing to its complicated structure, industralization is difficult; 2) during the detection, the flowing speed of liquid requires some sophisticated instruments, e.g. electronic osmosis device and etc.; 3) after reaction, some test results such as in fluorescence-labeled detection, cannot be read directly by a common biochip scanner, for the probe molecules are fixed on the inner surface.

Although there are many studies concerning the microchannel recently (e.g. No.98805243.1, 98813487.X and 98813488.8 in Chinese Patent Application; No.878437, 801390 and 090840 in USA Patent Application), a channel structure that can be easily manufactured at low cost is still one of heated topics in chip development. This invented channel is produced through immobilizing nano-structures or/and active nano-structures onto the support. For instance, nano-particles or/and active nano-particles are employed to form a coating in the position of channel on the substrate. Thus dimensions of the channel can be easily controlled, e.g. a microchannel or a wider channel can be formed. Besides, since the nano-particles can be transformed as required, the channel is endowed with the capacity of delivering the flowing phase. Moreover, the cost for manufacture is very low.

The device considered the fifth facet of the invention includes one of following: ELISA plate, planar chromatography reagent strip, and device that includes matrix for separation such as chromatography column.

The sixth facet of the invention is to provide a marking system for quantitative or/and qualitative analysis. The system contains markers and one type of them at least is the active labeling reagent/nano-structure/molecular labeling material complex. The nano-structure includes nano-particles or/and the structures composed of nano-particles. The nano-particle is non-magnetic inorganic non-metal particle with a diameter 1-100nm. And it is not the labeling enhancement itself.

The invented ligand/ nano-particle / molecular labeling material complex can be either a mixture or purified substance. The purified substance contains one or more molecular labeling materials, one or more types of nano-particles, one or more active labeling reagents and optional blocking agents. With the invented marker, a wide range of targets can be labeled, such as peptide drugs and polypeptide interactive drugs. The invented marker can label nucleic acids and pharmaceutics interacted with nucleic acids. The invented active labeling reagent is used in markers that bind themselves to targets to realize the function, e.g.: antigens, antibodys, single-stranded or multi-stranded DNA, RNA, nucleotides, as well as viruses, cells or their compositions etc. In this aspect, this invented ligand / nano-particle / molecular labeling material complex is different from ligand-nano-particle complex, such as ligands that do not contain nano-supports-particle ligands (No.02137418.X, 02115771.5 and 01133527.0 of Chiese Patent Applications). Since the labeling material in the invented complex is a molecular labeling material (such as Rhodamine) rather than nano-particles, then nano particles are supports rather than labeling material; the support can be employed as one of means for preserving some active sites of active labeling reagents.

The invented ligand / nano-particle / molecular labeling material complex also differs from ligand - microsphere - fluorescent particle compound (No.02121391.7 of Chinese Patent Application), molecular labeling material-micrometer particle ligand (No.0211411903 of Chinese Patent Application), for the support in the invented complex is nano-particle (e.g. nanometer silica), rather than microsphere or microparticle of big size. Nano-particles usually have much higher dispersity and binding activity.

In the above-described marking system, there are many methods for preparing active reagent/nano-structure/molecular marking complexs. For example, combine one or several types of the ligands, one or several types of nano-particles, and one or several types of the molecular labeling material in accordance with the following patterns: the ligand is bound with the nano-particle and then to the molecular labeling material; the nano-particle is bound with the molecular labeling material and then to the ligand; the ligand is bound with the molecular labeling material and then to the nano-particle; the ligand is bound with the molecular labeling material and the nano-particle concurrently, as well as other ways based on the above combinations. The method of preparing the invented complex is simple and the produced complex is of good water-solubility and high sensitivity.

In the marking system considered the sixth facet of the invention, the non-magnetic inorganic non-metal particle includes the non-magnetic inorganic non-metal particle described in the nano-structure considered the primary facet of the invention. In the marking system of the invention, the non-magnetic inorganic non-metallic particles contained in the compound include oxide particles with a size 1-100nm, optimally 1-50nm, as well as the derivatives thereof. The oxide particles include silica, titanium dioxide, and alumina. The derivatives include derivatives containing derivative group or/and coating organic compound on the surface. The derivative groups include one or more of the following: amino-groups, aldehyde-groups, epoxy-groups, amino diazane, diethylaminoethyl, diethyl- (2-hydroxypropyl) aminoethyl, carboxymethyl, sulfopropyl, mercaptoethylpyridine, siloxanyl, thioalcohol and alkyl-groups. The functional organisms include one or more of the following: surface active agents including the types of polyvinylpyrrolidone and Tween, polyelectrolyte including polyamino acid, oleophilic organisms including polysiloxane, ion exchange polymers including dextran derivative, agarose derivative, cellulose derivative, polyacrylamide, and active materials including heparin natrium. The said microcarrier in the microcarrier-coated derivative includes the nano-particle described above.

In the marking system considered the sixth facet of the invention, the molecular labeling materials include one or several types of the following: fluorescent material, chemiluminescent material, chemiluminescent catalyst, colored metal salt, dye and pigment, such as one of several materials below: fluorescein, Rhodamine, alga protein, silver salt, enzyme, basic black, basic violet, amino black, Coomassie blue, crystal violet and etc.

The seventh facet of the invention is to provide a quantitative or/and qualitative analysis method, which includes using the analytic or/and separating device considered the fifth facet of the invention to capture, deliver or/and separate sample targets, and using the marking system of quantitative or/and qualitative analysis considered the sixth facet of the invention to execute the labeling. This method involves at least: employment of the active nano-structured support to fix the target and employment of the ligand/nano-particle/molecular marking complex to label target; or/and, employment of the nano-structured channel, or/and active nano-structured channel to deliver, or/and separate the reaction media. The fixing and labeling of targets could be carried out in at least 2 ways: 1) after possible targets in a sample are fixed in the above-described device, the marker is used to label the targets which have been bound onto the said active nano-structured support; or, 2) after possible targets in a sample are bound with the label, the combination is fixed in the device. Moreover, in the implementation examples of the test method of the invention, the test sensitivity of active labeling reagent/nano-structure/molecular labeling material complex improves with the increase of nano-particle concentration, which differs totally from the nano-particle effect observed in the said method of preparing active nano-structured support. Therefore, the nano-particle weight/volume concentration (g/ml) in the complex of active labeling reagent/nano-structure / molecular labeling material herein is over 1/30000, preferably over 1/1000, and optimally over 1/100, or nano-particle mole concentration is higher than 0.25nM, preferably higher than 7.48nM, and optimally higher than 74.8nM.

The eighth facet of the invention is to provide a kit for quantitative or/and qualitative analysis, which comprises the analytic or/and separating device considered the fifth facet of the invention, or/and the marking system of quantitative or/and qualitative analysis considered the sixth aspect of the invention. The detailed description of these materials can refer to the related paragraphs above.

The kit considered the eighth facet of the invention is the one used for analyzing polypeptide or/and polypeptide-related drugs, including chip kit, ELISA kit, or planar chromatography kit for analyzing polypeptides. For analyzing nucleic acid or/and nucleic acid-related drugs, the kit is the optimal selection.

The ninth facet of the invention is to provide a quantitative or/and qualitative assay method, which includes using the analytic or/and separating device considered the fifth aspect of the invention to capture, deliver or/and separate sample targets. This method at least involves the following steps: employment of the active nano-structured support to fix the target; or/and employment of the nano-structured channel, or/and active nano-structured channel to deliver, or/and separate the reaction media. The fixation of targets could be carried out through at least 2 ways: 1) possible targets in a sample are fixed into the above-described device; or, 2) the possible targets in a sample are bound with the label, and then the combination is fixed into the device.

The tenth facet of the invention is to provide a kit for quantitative or/and qualitative analysis, which comprises the analytic or/and separating device considered the fifth facet of the invention. The detailed description of these devices can refer to the paragraphs above. The detailed description of the device can refer to the related paragraphs above.

The kit considered the tenth facet of the invention could be the kit used for analyzing polypeptide or/and polypeptide-related drugs, including chip kit, ELISA kit, or planar chromatography kit for analyzing polypeptides. For analyzing nucleic acid or/and nucleic acid-related drugs the kit is the optimal selection.

The eleventh facet of the invention is to provide a quantitative or/and qualitative assay method, which includes using the marking system of quantitative or/and qualitative analysis considered the sixth facet of the invention to execute the labeling. The method at least involves employing the marker to label targets captured by the active support, or adding the marker to a sample to label possible targets. Besides, in implementation examples about the test method of the invention, the test sensitivity of active labeling reagent/nano-structure/molecular labeling material complex improves with the increase of nano-particle concentration, which differs completely from the observed effect of nano-particle in the said method of preparing active nano-structured support. Therefore, the nano-particle weight/volume concentration (g/ml) of the complex of active labeling reagent/nano-structured/molecular labeling material is over 1/30000, preferably over 1/1000, and optimally over 1/100, or nano-particle mole concentration is higher than 0.25nM, preferably higher than 7.48nM, and optimally higher than 74.8nM.

The twelfth facet of the invention is to provide a kit for quantitative or/and qualitative analysis, which comprises the marking system of quantitative or/and qualitative analysis considered the sixth facet of the invention. The detailed description of the marking systems can refer to the above relevant paragraphs.

The kit considered the twelfth facet of the invention can be used for analyzing polypeptide or/and polypeptide-related drugs, including chip kit, ELISA kit, or planar chromatography kit for analyzing polypeptides. For analyzing nucleic acid or/and nucleic acid-related drugs the kit is the optimal selection.

The thirteenth facet of the invention is to provide a chip testing method, which includes at least the following steps:
(1) Prepare the sample, chip and chip marking system, wherein the said sample may contain targets; the said chip comprises immobilized active reagent arrays; the said chip marking system comprises labels, colorants, and optional color-controlling and color-setting agents. Moreover the said marker comprises the complex of active labeling reagent/nano-structure/catalyst, wherein the said nano-structure includes nano-particle or/and the structure composed of nano-particles; the said nano-particles are inorganic nano-particles, organic nano-particles or/and their derivatives with a particle size 1-100nm, wherein the inorganic nano-particles include non-magnetic inorganic nano-particles and magnetic inorganic nano-particles; the non-magnetic inorganic nano-particles include non-magnetic metal nano-particle and non-magnetic non-metal nano-particles; the derivatives include those bound with surface groups or/and coated organic compounds;
(2) Use immobilized active reagent to fasten the said target, and then immobilize the said nano-structure of the marker and the catalyst thereon through reaction between the target and the active labeling reagent in the marker; or
(3) Make the said target bound partially or completely with the said marker, and then immobilize the said nano-structure of the marker and the catalyst thereon through reaction between the target and the immobilized active reagent;
(4) Add the said colorant and proceed with the coloration, under the action of catalyst, on the said nano-structure rather than on the link between the said target and the immobilized active reagent, wherein the coloration of visible light includes optional use of color-controlling agent and color stabilizer;
(5) Test and analyze the results of coloration (step 4) , and the coloring results (step 3 and 4).

The technical design of the invented method differs from that of CN1351712T in patent application, for the latter executes the catalytic reaction at the link of the active labeling reagent-captured targets; therefore it is difficult to control the reaction velocity. By contrast, the invented method executes the catalytic reaction on the nano-structure that is bound to the captured targets; therefore it is easy to control the nano-structure parameters (the volume of active labeling reagent, the volume of catalyst, the size of nano-particle etc.) and the specificity (such as the tendency of easy separation etc.) so as to control the reaction velocity. As a result, the invented method has faster target-marker reaction time and higher testing ability for specificity.

In the method considered the thirteenth facet of the invention, the said marker also comprises stain, which reacts prior to, during or/and after the said coloration, wherein the stain exists in one or more of the following markers: complex of active labeling reagent /nano-structure / catalyst/ stain, complex of active labeling reagent / nano-structure/ stain, and complex of active labeling reagent/stain, wherein the nano-structure can be the one described in above facets of the invention. The method of the invention is an optimal design, executing both coloring and dyeing in order to obtain higher sensitivity and specificity.

In the method considered the thirteenth facet of the invention, the said nano-particle in the marking system includes the nano-particle described in the nano-structure considered the primary facet of the invention.

In the method considered the thirteenth facet of the invention, the catalyst includes metal catalyst or/and enzyme catalyst in the reduction reaction of metal compound; the coloring agent includes dyes, and colorant includes metal compound.

The fourteenth facet of the invention is to provide a chip testing method, which at least includes the following steps:
a) Prepare the sample, chip and chip marking system, wherein the sample may contain targets; the chip comprises immobilized active reagent arrays; the chip marking system comprises markers, colorants, and optional color-controlling agents and color stabilizers, wherein the markers comprise active reagent, catalyst, stain, and optional nano-structures, wherein the nano-structure, stain, catalyst, colorant are the nano-structure, stain, catalyst, colorant respectively in the chip marking system of the invention described above;
b) Add the sample to the chip. After the reaction between the immobilized active reagent and the possible targets, add the marker and colorant and proceed with the dyeing and coloring at the reaction site; or
c) Mix the sample with the marker partially or completely, load the mixture onto the chip after the reaction between the active labeling reagent and the possible targets, add the colorant after the reaction between immobilized active reagent and the possible targets, and then proceed with the dyeing and coloring at the reaction site of the chip;
d) Test and analyze the results of dyeing and coloring in step (b) or (c).

The fifteenth facet of the invention is to provide a chip kit that comprises marking systems, in which there is at least one that is the marking system of this invention described in the above-mentioned paragraphs. See the related paragraphs above for details.

The kit considered the fifteenth facet of the invention can be used for analyzing polypeptides or/and polypeptide-related drugs. It is the optimal selection for analyzing nucleic acid or/and nucleic acid-related drugs.

The sixteenth facet of the invention is to provide a test method of polypeptide analysis-chip, which includes at least one of the following steps:
(a) Mix the sample with magnetic nano-particle;
(b) Mix the sample with the complex of active reagent/magnetic nano-particles;
(c) Make the sample contact and react with the chip. During the reaction, the external magnetic field is optional, wherein the said chip is a nano-structured chip, wherein the nano-structure comprises magnetic nano-particles;
(d) Apply the compound of active labeling reagent / magnetic nano-particle /molecular labeling material to the labeling reaction. During the labeling process, the external magnetic field is optional, wherein the compound of active labeling reagent / magnetic nano-particle / molecular labeling materia is the mixed or purified material containing one or several types of molecular labeling material, one or several types of magnetic nano-particles, one or several types of active reagents as well as optional blocking agents.

In this method, the magnetic nano-particle has at least one-dimensional size 1-200nm, preferably 1-100nm, and optimally 1-50nm. Moreover it is neither magnetic particle nor its derivatives used as enhancers for molecular labeling material. The active reagent is selected from the materials interacted with polypeptides in the following list: polypeptides, polysaccharides, vitamins, antibiotics, viruses, cells and functional organisms. The concentration of the magnetic nano-particle in the complex of active reagent/magnetic nano-particle/molecular labeling material in the process of labeling is 1/30000(g/ml), or over 0.25nM mole concentration; preferably over 1/3000(g/ml) or 2.4 nM mole concentration; and optimally over 1/500(g/ml), or 15.0nM mole concentration;

In the method considered the sixteenth facet of the invention, the magnetic particle is selected from ferrite and its derivatives including ferriferous oxide and iron trioxide, wherein the derivatives include those bound with surface groups or/and coated with functional organisms.

In the method considered the sixteenth facet of the invention, the external magnetic field is in pulsed wave mode.
The seventeenth facet of the invention is to provide a kid of polypeptide analysis-chip that comprises the magnetic nano-particle described by the above facets of the invention. See the related paragraphs above for details. In the kit, the magnet nano-particle is used alone to generate the mixture of reaction media; the said ligand/magnetic nano-particle is used to concentrate the sample target, the said complex of ligand/magnetic nano-particle/molecular labeling material is used to label the reaction. It also includes the use of the separating device considered the fifth facet of the invention.

The invented nano-structured support used in separation or/and analysis has advantages of manufacturing simplicity, low cost and good adsorbability.
The invented method of preparing the nano-structured support has the following advantages: simplicity, efficacy and low cost, and an obtainable stable nano-structured support with specific features of nano-structure without heating the object up to its sintering point (thus with less change in the support).

The analytical or/and separating devices of the invention has the advantages of high sensitivity, manufacturing simplicity, low cost, high reaction efficiency and high reaction. Velocity.
The method of preparing the active nano-structured support of this invention has the following advantages: high adaptability, simplicity, efficacy, low cost and an obtainable stable nano-structured support with specific features of nano-structure without heating the object up to its sintering point (thus with less change in the support)

The invented analytic or/and separating device of the invention has the following advantages: high sensitivity, simplicity in preparation, low cost, high reaction efficiency and high reaction velocity.

The marking system of quantitative or/and qualitative analysis of this invention has the advantages of high reaction efficiency and simplicity in preparation.

The invented test method (which makes use of the complex of active nano-structured support and ligand/nano-particle/molecular marking material) has the following advantages: utilizing both active nano-structured support to capture target polypeptides in the sample, and the complex of ligand / nano-particle / molecular labeling material to label, which thereby raises greatly the test sensitivity or/and velocity.
The invented test kit (containing the active nano-structured support and the complex of ligand/nano-particle/molecular marking material) integrates the merits of both active nano-structured support and complex of ligand/nano-particle/molecular marking mateiral, and therefore enjoys the high sensitivity, high testing velocity and simple preparation.

The invented test method (which makes use of the active nano-structured support) has the advantages of high sensitivity, high testing velocity and simple preparation. The invented test kit (which contains the active nano-structured support) has the advantages of high sensitivity, high testing velocity and simple preparation.

The invented test method (which makes use of the complex of ligand/nano-particle/molecular marking material) has the advantages of increased test sensitivity or/and test velocity.

The invented test kit (which contains the complex of ligand/nano-particle/molecular marking material) has the advantages of increased test sensitivity or/and test velocity.

The invented chip test method (which contains the complex of ligand/nano-particle/molecular marking material) has the advantages of increased test sensitivity or/and test velocity.

The invented chip test method (which makes use of the complex of active labeling reagent /nano-structure/catalyst has the advantages of increased test sensitivity, test velocity and test specificity.

The invented chip kit (which contains the complex of active labeling reagent /nano-structure/catalyst) has the advantages of increased test sensitivity, velocity and test specificity.

The invented chip test method (which makes use of markers including active reagent, catalyst and stain) has the advantages of increased test sensitivity and specificity. The invented chip kit (which contains markers including active reagent, catalyst and stain) has the advantages of increased test sensitivity, velocity and specificity.

The invented chip kit (which contains the complex of active nano-structured support and/or ligand /nano-particle/molecular marking material) has the advantages of increased test sensitivity or/and velocity.

The invented test method of polypeptide analysis-chip (which makes use of magnetic nano-structure) has the advantages of increased test sensitivity or/and velocity.
The invented polypeptide analysis-chip kit (which contains magnetic nano-structures) has the advantages of increased test sensitivity or/and velocity.
The separation method of the invention has the advantages of high adsorbability and high resolution.

### Illustration

Picture 1 is the DFM plane figure of a nano-structured support
Picture 2A and 2B are respectively the DFM plane figure and stereogram of an active nano-structured support.
One thing that needs explanation is these pictures are only few of many DFM plane figures about the nano-structured support and active nano-structured support. Therefore, it cannot be taken as the representative of other nano-structured supports and active nano-structured supports of this invention.

### Detailed description of the invention implementation

### Definition of the terms

The term "test device" in the invention refers to items containing ligand which is employed to react with target in a sample in the course of quantitative or/and qualitative analyses, e.g. instruments for capturing ligand, consumables and labeling kits used for capturing and labeling ligand. Cases at concern are analysis-chip, ELISA plate, affinity electrophoresis strip, affinity chromatography column, planar chromatography reagent strip, analysis-chip kit, ELISA plate kit, affinity electrophoresis kit, etc. The course of quantitative or/and qualitative analyses can be executed in vitroor in vivo routes.

The term "separation device" in the invention refers to separation necessities containing materials with separating functions used in the course of separation. The course of separation is such that the whole or parts of sample components are obtained via separation methods. Chromatography device is an example for such a device. Chromatography support etc. is an illustration for materials with separating functions.

The term "ligand/magnetic nano-particle/support" in the invention refers to a complex containing at least ligand, magnetic nano-particles and supports, wherein the ligand, magnetic nano-particles and supports can be combined in various fashions including direct and indirect bindings.

The term "active reagent" in the invention refers to materials used for capturing targets through interaction (such as affinity effect, ion exchange, oleophilic effect, etc.). It includes ligand and ion exchanger, such as: diethylaminoethyl (DEAE), diethyl- (2-hydroxypropyl) aminoethyl (QAE), carboxymethyl (CM), sulfopropyl (SP), mercaptoethylpyridine (MEP), -NR3+, -RCOOH, siloxanyl, thioalcohol, alkyl groups, antigens, antibodies, ligands, ligation-philic molecules screened by the evolving technology of ligand exponent-enhancing system, ligand, polypeptides, polysaccharides, coenzymes, cofactor, antibiotics, steroid, viruses, cells etc.

The term "labeling active reagent" in the invention refers to the materials in the marker, used for labeling the targets through the interaction (including affinity effect, ion exchange, oleophilic effect, etc.), including ligate, such as: antigens, antibodies, ligand, ligation-philic molecule screened by the evolving technology of ligand exponent-enhancing system, ligand, polypeptides, polysaccharides, coenzymes, cofactors, antibiotics, steroid, viruses, cells etc.

The term "polypeptides" in the invention includes natural or synthetic proteins, protein fragments, synthetic peptides, etc.; the normal targets in immunoassay and the popular ligands in detection, e.g. antigens, antibodies, etc. all belong to polypeptides.
The term "nano-particles" in the invention refers to particles of the support, which, in three-dimensional space, have at least one-dimensional size less than 500nm, preferably less than 100nm, optimally less than 50nm.

The term "support" in the invention includes the conventional support and nano-structured support, wherein the conventional support is the one which has no immobilized nano-structure there on the surface; the nano-structured support is the one which has the immobilized nano-structure thereon. The support includes the planar supports (e.g. substrates of biochip, ELISA plate etc), granular supports (e.g. the chromatography gel, especially the micrometer particle chromatography gel) and membranous supports (e.g. plane chromatography strip).

The term "substrate" in the invention refers to the support with immobilizing function, made up of macroscopic planes on one side, e.g. the substrate of analysis-chips, the substrate of ELISA plates, electrophoresis gel film, plane chtomatography supports etc.

Term "complex of ligand / nano-particle / substrate" in the invention refers to one kind of composition containing ligand, nano-particles and substrates, whose bonding can be in various forms, such as ligand-nano-particle-substrate, ligand-nano-particle-ligand-substrate, ligand-nano-particle-ligand-nano-particle-substrate, ligand-nano-particle-ligand-nano-particle-ligand- substrate etc.

The term "active nano-particle " in the invention refers to the complex of active reagent and nano-particles by the covalence or/and non-covalence bond.
The term "reactor" in the invention refers to the location (where active support is fixed, and the specific reaction takes place with the target molecule,) and other relevant collected structures thereto, such as the reaction-well, relevant partition structure, liquid inlet and outlet etc., the well on 96-well ELISA plate, reagent strip of rapid test kit etc.

The term "baseplate" in the invention refers to the product that is based on the substrate, combined with other optional structures (e.g. partition structure), and used for generating the chip after it is fixed with the ligand. There are one or several substrate-wells on the baseplate. Usually, there is no partition structure on the single-substrate-well baseplate, which is the substrate in such a case (e.g. amino-group glass slide available on market). There are partition structures on the baseplate of multi-substrate-well; in this case the baseplate includes substrates and partition structures. The substrate-well fixed with probes will become a reactor, while the substrate with multi-substrate-well can form a chip with multiple reactors. The substrate is used for immobilizing ligand and other auxiliary (if required), the chemical and optical properties on its surface are crucial factors to decide the performance and cost of the chip.

The term "substrate-well" in the invention refers to the structure made up of substrates and the partition structures thereof.
The term "analysis-chip" or "chip" in short in the invention refers to a test device for qualitative and/or quantitative analysis (including, but not confined to, biochip, microarray, bioarray in English). In its reactor, the result of specific reaction between the microligand and target molecule in the sample can be identified in an addressable way. The core of chip is the reactor thereof, and core of reactor is the substrate of the chip and ligand immobilized thereon. The Chip includes microchannel chips and microarray chips (equivalences of biochips, microarray, bioarray in English), but as known to all it doesn't include existing rapid test strips. The chip in the invention consists of single-reactors or multi-reactors with an optional marking system. In the reactor the distribution density of ligand on the substrate is more than 10 spot/cm², optimally more than 20 spot/cm², while the spot area for each ligand is not larger than 1mm². Featured by its high-flux and miniaturization, biochips have a wide application, including gene expression determination, genes screening, drugs screening, diseases diagnosis and treatment, environment surveillance and control, legal identification, etc. Sensitivity and specificity are the two important quality indicators in biochip detection, which, however, is largely determined by the support, especially the active support

The term "nano-structured chip" in the invention refers to a region containing at least one of the said active nano-structure (e.g. a spot in the probe microarray). A chip can have many reactors, and a reactor can have many spots (probe spots), which contain active reagent. If one of the spots is the active nano-structured region of the invention, then the chip is regarded as the invented active nano-structured support or nano-structured chip.
Term "chromatography "in the invention includes affinity chromatography, reversed phase chromatography, hydrophobic chromatography, ion exchange chromatography, etc. It can be classified as planar chromatography (e.g. rapid test strip and rapid test kit) and column chromatography etc.

The term "molecular labeling material" in the invention refers to materials used to generate or help generate the test signal, which are in the form of molecule while labeling, e.g. the commonly used markers in the chip detection like Rhodamine, Cy3, Cy5 and etc.

The term "nano-structure" in the invention refers to structures that possess the nanometer size and have partial or complete nanometer effects (e.g. surface effect, size effect).

The term "convex distance" in the invention refers to the distance from top to bottom of a convex object.

The term "cross-section at half height of a convex object " in the invention refers to the horizontal plane of a convex object at the half height of the convex distance.

The term "mono-active support" in the invention refers to an active support with only one type of active reagent immobilized thereon.

The term "multi-active support" in the invention refers to an active support with several types of (more than one type of) active reagents immobilized thereon.

The term "nano-particle mole concentration" in the invention refers to the mole concentration when nano-particles are taken as molecule, which indicates the number of nano-particles in unit volume / liquid. It is calculated as follows: count the number of nano-particles on the basis of nano-particle concentration, average radius and density provided by the current suppliers, and then take the numbmer as that of molecule into the calculation of the number of nano-particles in unit volume / liquid according to the known formula of mole concentration-weight/volume concentration (g/ml).

Following implementation examples will offer more detailed explanations for this invention.

### Implementations

The nano-particles and the derivatives used in the following implementation examples are showed in Table 1.

**Table 1**

| | | | |
|---|---|---|---|
| Nano-particle | The size of Nano-particle | Introduced group | Manufacturer |

| 1. Oxide nano-particle & derivative | | | |
|---|---|---|---|
| Silica nano-particle (STN-3) | 15-25nm | - | Zhejiang Zhoushen MingRi Nanomaterials Co., Ltd. |
| Silica (LUDOX AS-40) | 30-40nm | - | Sigma-Aldrich Corp. |
| Titanium oxide nano-particle | <80nm | - | Zhejiang Zhoushen MingRi Nanomaterials Co., Ltd. |
| Hydrophobic silica nano-particle (CDS7) | 25-35nm | Alkyl group | Zhejiang Zhoushen MingRi Nanomaterials Co., Ltd. |

| Metallic nano-particle & derivative | | | |
|---|---|---|---|
| Colloidal gold | 20-30nm | - | Fujiang Quanzhou Changli Biochemical Co., Ltd. |
| Amino colloidal gold (2020) | 1.4nm | Amino group | American NanoProbes Co. |

| 3. Magnetic nano-particle | | | |
|---|---|---|---|
| Water-base magnetic solution (NG-21A) | 10-50nm | | Mianyang Biyang Magnetic Material Science Co. |

The active reagents used in the following implementation examples are showed in Table 2.

**Table 2**

| Active reagent | Type | Source |
|---|---|---|
| EBV-VCA-P18 antigen | Synthetic peptide | Self-prepared* |
| Hepatitis C virus antigen (HCV AG) | Protein | Institute of Hepatology Beijing university |
| Acquired immunodeficiency syndrome virus antigen (HIV AG) | Protein | Institute of Hepatology Beijing university |
| Syphilis antigen | Protein | Institute of Hepatology Beijing university |
| Hepatitis B virus surface antibody (HBsAb) | Protein | Institute of Hepatology Beijing university |

| | | |
|---|---|---|
| • For the preparing method, please refer to Tranchand-Bunel, D., Auriault, C., Diesis, E., Gras-Masse, H. (1998) Detection of human antibodies using "convergent" combinatorial peptide libraries or "mixotopes"designed form a nonvariable antigen: Application to the EBV viral capsid antigen p18, J. Peptide Res. 52, 1998, 495-508. | | |

Substrates and micrometer particles used in the following implementation examples are showed in Table 3.

**Table 3**

| Substrate | Surface polymer | Derivative group | Manufacturer |
|---|---|---|---|
| 1. Chip substrate | | | |
| Glass slide | - | - | American TeleChem International, Inc. |
| Amino group glass- slide | - | Amino group | American TeleChem International, Inc. |
| Aldehyde group glass- slide | - | Aldehyde group | American TeleChem International, Inc. |
| Epoxy group glass- slide | - | Epoxy | American TeleChem International, Inc. |
| Amino diazane glass- slide | - | Amino diazane | Self-prepared |
| PVP-coated glass- slide | PVP | PVP | Self prepared** |
| DEAE dextran-coated glass- slide | DEAE dextran | DEAE | Self-prepared ** |
| QAE dextran-coated glass- slide | QAE dextran | QAE | Self-prepared ** |

| 2. The substrate of ELISA plate | | | |
|---|---|---|---|
| 96-well polystyrene plate | | | Shenzhen Jincanhua Industrial Co., Ltd. |

| 3. Plane chromatography reagent strip | | | |
|---|---|---|---|
| Nitrocellulose film strip | | | Fujiang Quanzhou Changli Biochemical Co., Ltd |
| Nylon cellulose film strip | | | Fujiang Quanzhou Changli Biochemical Co., Ltd |

| 4. micrometer particle | | | |
|---|---|---|---|
| Silica gel (particle size of 40-60µm) | | | Chemical Institute, Chinese Academy of Sciences |
| Sephadex A50 | | | Pharmacis Corp. |

| | | | |
|---|---|---|---|
| *: For the details of the preparing method, refer to Melnyk O et al, Peptide arrays for highly sensitive and special antibody-binding fluorescence arrays, Bioconjug Chem.13: 713-20.2002 | | | |
| **: For the tails of the preparing method, refer to China Patent Application Number 03135618.4 | | | |

### Implementation example 1: the preparation of nano-particle derivatives

The nano-particle derivatives prepared herein are coated with high polymer with the function of organic matter. Nano-particles employed include those listed in Table 1 (the said nano-particles are all non-crystal nano-particles). Functional polymers employed are listed in Table 2, including polyionic organic matter (polylysine), ionic derivative polymers (DEAE-dextran, QAE-cellulose, amino diazane-polylysine), and high polymer surfactant (polyvinylpyrrolidone).

The keys points of preparing organic-matter-coated nano-particles are as follows: put nano-particles in ultrasonic vibration, set them apart into different solution of nanoparticle in accordance with different concentration, mix the solution with the same volume of organic solution whose concentration is 1/12500 (w/v), make it react in ultrasonic vibration at 37°C for 1 hour , instill the resultant into a rotating tube with chromatographic gel, centrifuge it under the condition of 4000 rpm/min, remove the liquid from the collecting tube and make it ready for use (when all conditions are optimized, the process of centrifugal separation can be omitted).

Nano-particle-coated derivatives prepared in the implementation example are listed in Table 4.

**Table 4**

| Nano-particle derivative | Nano-particle | Coating material | |
|---|---|---|---|
| | | Functional high polymer | Manufacturer |
| DEAE-dextran-coated nano-particle | Silica (LUDOX AS-40) | DEAE-dextran | Pharmacia Corp. |
| QAE-cellulose-coated nano-particle | Silica nano-particle (STN-3) | QAE-cellulose | Chengdu Chenguang Chemical Industry Research Institute |
| CM―dextran-coated nano-particle | Silica (LUDOX AS-40) | CM―dextran | Pharmacia Corp. |
| Polyvinylpyrrolidone-coated nano-particle | Silica (LUDOX AS-40) | Polyvinylpyrrolid one | Tianjin Jinyu Fine Chemical Co., Ltd. |
| Polylysine-coated nano-particle | Silica nano-particle (STN-3) | Polylysine | Sigma Corp. |
| Amino diazane-polylysine-coa ted nano-particle | Silica nano-particle (STN-3) | Amino diazane-polylysin e | Chengdu Chempep New Technology Co., Ltd |

### Implementation example 2: the preparation of active nano-particles

Active nano-particles either prepared or used in this implementation example include those selected from Table 1 and nano-particle derivatives prepared in Implementation example 1, while the employed active reagents are selected from the ligands in Table 2.

The preparation of active nano-particles in this implementation example includes the following steps: put nanoparticles or nanoparticle polymers in ultrasonic vibration, prepare them dispersively into nano-particle solution with concentration ranging from 1/6250 to 1/25000 (w/v), mix the solution with ligand solution whose concentration is 1-2 mg/ml (the mixture ratio is 1:1) and make the mixture react for 1 hour at room temperature. If purification is necessary, instill the resultant solution into a rotating tube with chromatographic gel, centrifuge it under the condition of 4000 rpm/min, remove it from the collecting tube and make it ready for use. The same method can also be used for preparing active nano-particles which make use of either other nano-particles or active reagents. The acquired active nano-particles prepared in this example are showed in Table 5.

**Table 5**

| Functionalized nano-particle | Nano-particle | Functional reagent (ligand) |
|---|---|---|
| EBV antigen-STN-3 | Silicon oxide nano-particle (STN-3) | EBV-VCA-P18 antigen |
| HCV Ag-STN-3 | Silicon oxide nano-particle (STN-3) | HCV Ag |
| HIV Ag -STN-3 | Silicon oxide nano-particle (STN-3) | HIV Ag |
| HBs antigen -STN-3 | Silicon oxide nano-particle | HBs antigen |
| | (STN-3) | |
| EBV antigen - Ludox | Silicon oxide (Ludox As-40) | EBV-VCA-P18 antigen |
| HCV Ag - Ludox | Silicon oxide (Ludox As-40) | HCV Ag |
| HIV Ag - Ludox | Silicon oxide (Ludox As-40 ) | HIV Ag |
| HBs antigen - Ludox | Silicon oxide (Ludox As-40) | HBs antigen |
| HBs antibody -Ludox | Silicon oxide (Ludox As-40 ) | HBs antibody |
| HCV Ag-titanium dioxide | Titanium dioxide nano-particle | HCV Ag |
| HIV Ag-titanium dioxide | Titanium dioxide nano-particle | HIV Ag |
| HCV Ag -CDS7 | Hydrophobic silicon nano-particle (CDS7) | HCV Ag |
| HIV Ag -CDS7 | Hydrophobic silicon nano-particle (CDS7) | HIV Ag |
| EBV antigen -colloidal gold | Colloidal gold | EBV VCA-P18 antigen |
| EBV antigen-amino-colloidal gold | Amino -colloidal gold | EBV VCA-P18 antigen |
| HCV Ag -DEAE particle | DEAE-dextran-coated nano-particle | HCV Ag |
| HIV Ag-DEAE particle | DEAE-dextran-coated nano-particle | HIV Ag |
| HCV Ag-QAE particle | QAE- dextran-coated nano-particle | HCV Ag |
| HIV Ag -QAE particle | QAE- dextran-coated nano-particle | HIV Ag |
| HCV Ag-polyvinylpyrrolidone particle | Polyvinylpyrrolidone-coated nano-particle | HCV Ag |
| HIV Ag-polyvinylpyrrolidone particle | Polyvinylpyrrolidone-coated nano-particle | HIV Ag |
| HCV Ag -polylysine particle | Polylysine -coated nano-particle | HCV Ag |
| HIV Ag - polylysine particle | Polylysine -coated nano-particle | HIV Ag |
| HCV Ag- amino diazane lysine particle | Amino-diazane polylysine-coated nano-particle | HCV Ag |
| HIV Ag amino diazane lysine particle | Amino-diazane polylysine-coated nano-particle | HIV Ag |

### Implementation example 3: preparation of nano-structured support

The nano-structured support prepared in this example includes the complex of nano-particles / substrates and complex of nano-particles / micrometer particles. In accordance with the patented preparation, nano-particle / micrometer particle /substrate complex can also be prepared. The nano-structured support prepared in this example can be used as substrates of analysis-chip, ELISA plates, planar chromatography reagent strips, and chromatography gel respectively.

The supports used in the implementation example are conventional supports, including glass slides, 96-well polystyrene plates and nitrocellulose membrane strips in Table 1. The said preparation is applicable to conventional supports made of the following materials or the derivatives thereof: silica, silica gel, ceramics, metallic oxide, metal, other polymer material and their compounds. The nano-particles used in this example include inorganic nano-particles (e.g. silica nano-particle (STN-3), silica (LUDOX AS-40), titanium dioxide nano-particle and colloidal gold in Table 1) and their derivatives (e.g. substance prepared in this implementation example). The said method is also applicable to preparations of organic nano-particles and other inorganic nano-particles, such as: inorganic non-magnetic oxide nano-particles including aluminum oxide particles, non-magnetic metal nano-particles including particles of gold, vanadium, and lead, organic nano-particles including particles of plastic, polysaccharide, emulsion and resin. The nano-particle derivatives used in this example include those bound with surface group or/and coated with organic matter. The surface groups include amino-group and the coating organic matter includes polyvinylpyrrolidone and dextran derivative.

The glass slide in this example is coated with derivatives, where the surface groups include amino, aldehyde, epoxy and amino diazane. Coating organic compounds include polyvinylpyrrolidone and dextran derivatives. The method in this example is also applicable to other derivatives of nano-particles or/and supports, e.g. those with the following surface groups or/and coating organic compounds. The surface groups include one or more types of the following: diethylaminoethyl, diethyl-(2-hydroxypropyl) aminoethyl, carboxymethyl, sulfopropyl, mercaptoethylpyridine, siloxanyl, thioalcohol and alkyl group. The coating organic compounds include one or more types of organisms below: surface active agents including the types of Tween, polyelectrolyte including polyamino acid, oleophilic organisms including polysiloxane, ion exchange polymers including dextran derivative, agarose derivative, cellulose derivative, polyacrylamide, and affinity materials including heparin natrium, antigen and antibody.
1) The preparation of nano-particle/substrate complex
   (1) The preparation of chip substrates by immobilization of nano-particles in the form of nano-particle suspensions
      (a) Direct immobilization
         Soak the slides and other chip substrates in Table 1 in the nano-particle suspensions of different concentration selected from Table 3 or 4 for over 2 hours, and then rinse and dry them. Numerous nano-structured substrates of chip can be acquired via this method.
      (b) Immobilization after activation
         Activate nano-particles or/and substrates first and then immobilize them. For example, nano-particles will undergo activation first, which will help them to bind up with active reagents or/and supports. Then their suspension will be put into contact with the supports so as to have immobilization reaction; or the supports will undergo activation first, which will help them to bind up with the nano-particles or/and the active reagents. Then they will be put into contact with the nano-particle suspension so as to have immobilization reaction.
         For example, the slides and other chip substrates in Table 1 are soaked in the nano-particle derivative suspensions of different concentration prepared in example 1 (Table 4) for over 2 hours, and then rinsed and dried. In another case, the slides in Table 1 are treated with 10% concentration of NaOH and HNO3, and then cleaned and dried as activated slides, which are then soaked in the nano-particle suspensions of different concentration selected from Table 3 or 4 for over 2 hours, then cleaned and dried. Numerous nano-structured chip substrates can be acquired via this method.
      (c) Immobilization before activation
         Put the nano-structured suspension into contact with the support and obtain immobilization reaction first, and then proceed with activation process, which helps immobilize the active reagent. For example, the slides in Table 1 are soaked in the nano-particle suspensions of different concentration selected from Table 3 or 4 for over 2 hours, cleaned and dried afterward and then activated with the method of slide activation. Numerous nano-structured substrates of chip can be acquired with this method.
   (2) Preparation of chip substrates through chemical cross-linking method
      Employ chemical cross-linking method to increase the stability of high-polymer-coated nano-particles immobilized on the support. For example, coat the slides in Table 1 with DEAE-dextran, then soak them in CM-dextran-coated-silica nano-particle suspension of different concentration prepared in example 1 for over 2 hours, have them rinsed and dried and finally cross-link them with dextran by cross-linker epoxy chloropropane. Numerous nano-structured chip substrates of high stability can be acquired via this method.
   (3) Preparation of chip substrates through heat treatment method
      Employ heat treatment to increase the stability of nano-particles immobilized on the support. For example, heat the chip substrate prepared by the immobilization of nano-particle suspension in a temperature range between 30 °C and the sintering point of the support and then cool it down, which can increase its stability of immobilization. In comparison with the substrate prepared by immobilization of nano-particle suspension only, the chip substrate prepared by using heat treatment (e.g. 37°C over 15 hours, 60°C over 10 hours, or 150 °C over 1 hours) presents higher immobilization intensity in Rhodamine-labeled secondary antibody after both being rinsed supersonically for 1 hour. Numerous nano-structured chip substrates of high stability can be acquired through this method.
   (4) The preparation of chip substrates with nano-structured channels
      The nano-structured region in the invented nano-structured support can be used as transporting channels for fluids. The preparation of chip substrates with nano-structured channels in the implementation example is similar to that of chip substrates mentioned above. However, the chip substrate of this method will not be soaked in the nano-particle suspension. Instead, according to the size and position of the channels on the substrate, the nano-particle suspension of different concentration, in a fixed quantification and orientation (e.g. to demarcate the substrate surface with lines whose width is 0.3-1.0mm, and length 3mm), is painted onto the substrate surface for reaction for more than half an hour. After that, it is rinsed and dried. The hydrophilic nano-particles immobilized onto the substrate of glass slides (e.g. LUDOX AS-40 in Table 1, DEAE-dextran-coated nano-particles, QAE-cellulose-coated nano-particles and polyvinylpyrrolidone-coated nano-particles in Table 4) will form a nano-structured region, whose surface-water contact angle is, as measurement proves, smaller than that of substrate of glass slides, and therefore of water-drainage function. Though with no intention getting into any theoretical discussion, we would like to say that the invented micro-channel owing to the nano-particle effect thereon (e.g. even larger surface area, more tiny concave objects) is the cause or partially the cause of this kind of "capillary action". Although the micro-channel is simple, professionals all know that a variety of micro-channel chips will become available if the said micro-channel is combined with other well-known techniques in micro-channel chip preparation.
   (5) The preparation of nano-particle coated micro-plates
      The substrate used in this implementation example is a 96-well polystyrene plate (Shenzhen Jincanhua Industrial Co., Ltd). The employed nano-particles are colloidal gold, silica nano-particles (STN-3) and silica (LUDOX AS-40) (See Table 1) respectively. The said nano-particle coated micro-plates in the implementation example is prepared as follows: make nano-particle solutions of different concentration contact with the bottom of polystyrene micro-plates, proceed with the reaction at room temperature for 15 hours, and then rinse the plate repeatedly with distilled water.
      The above-mentioned chemical cross-linking method and heat treatment method can also be used for preparing nano-particle coated micro-plates. Some nano-particle-coated micro-plates prepared in the implementation example are listed in Table 6.
   (6) The preparation of nano-particle-coated planar chromatography strips
      The key points of this preparation are the same as the preparation of the said nano-particle-coated micro-plates. Some nano-particle-coated planar chromatography strips prepared in the implementation example are listed in Table 6.
2) The preparation of nano-particle/micrometer particle complex
   The nano-particle/micrometer particle complex prepared in the implementation example includes the nano-particle/micrometer particle complex used for preparing the analysis-chip, ELISA plate, planar chromatography strip and chromatography gel. The micrometer particles in this example are those listed in Table 1, including conventional chromatography gel such as silica gel (particle size 40-60µm produced by the Chemical Institute, Chinese Academy of Sciences) and dextran (by Pharmacis Corp.). The preparing method in this example is also applicable to micrometer particles made of the following materials or the derivatives thereof: ceramics, metallic oxides, metals, other polymer materials and their compounds, and micrometer particles with nano-particles distributed on the surface.
   The nano-particles used in this example include inorganic nano-particles (e.g. Silica nano-particles (STN-3), Silica (LUDOX AS-40), Titanium dioxide nano-particles and Colloidal gold) and their derivatives (e.g. the prepared chemicals in the implementation example). The method in this example is also applicable to organic nano-particles and other inorganic nano-particles, such as: inorganic non-magnetic oxide nano-particles including the particles of alumina, the non-magnetic metal nano-particles including the particles of gold, vanadium and lead, the organic nano-particles including particles of plastic, polysaccharide, emulsion and resin. The nano-particle derivatives used in this example include those bound with surface groups or/and coated with organic compounds; the surface groups include amino-groups; the coating organic compounds include polyvinylpyrrolidone and dextran derivatives. In the derivatives of glass slides used in this example, the surface groups include groups of amino, aldehyde, epoxy and amino diazane; coating organic compounds include polyvinylpyrrolidone and dextran derivatives. The method in this example is also applicable to other derivatives of nano-particles or/and supports, e.g. those with the following surface groups or/and coating organic compounds: a) The surface groups include one or more of the following chemicals: diethylaminoethyl, diethyl- (2-hydroxypropyl) aminoethyl, carboxymethyl, sulfopropyl, mercaptoethylpyridine, siloxanyl, thioalcohol and alkyl group; b) The coating organic compounds include one or more of the following organisms: surface active agents like Tween types, polyelectrolyte like polyamino acid, oleophilic organisms like polysiloxane, ion exchange polymers like dextran derivative, as well as agarose derivatives, cellulose derivatives, polyacrylamides, and affinity materials like heparin natrium, antigens and antibodies.
   The key points in preparing nano-particle/micrometer particle complex are almost the same as those in the preparation of nano-particle/substrate complex mentioned above. The only difference is that micrometer particles are used here to replace the substrates. Part of the nano-particle/micrometer particle complex prepared in this implementation example is listed in Table 6.
3) Evaluation of nano-structured supports
   In this implementation example, nano-structure units and their distribution are ascertained by the scanning probe microscope (SPA-300HV, DFM) and electronic scanning microscope (See Picture 1). In the nano-structured supports prepared in this example, the nano-structure units are all arranged nonorientationally (See Picture 1).

In this implementation example, the height, the minimum size of nano-convex at its half height and the distribution density is measured by the scanning probe microscope (SPA-300HV, DFM) and the analytical software (See Picture 1). In the nano-structured support prepared in this example, when the concentration (w/v) of nano-particle suspension used for coating the support is 1/250 or 1/175000, the height is over 3nm, and the minimum size of nano-convex at its half height is 1-500nm at least in one-dimensional size, the distribution density of nano-convex is less than 10/µm². However, when the concentration (w/v) of nano-particle suspension ranges from 1/25000 to 1/50000, the height is over 3nm, and the minimum size of nano-convex at its half height is 1-500nm at least in one-dimensional size, the distribution density of nano-convex is more than 10/µm² , even more than 20/µm².

In this implementation example, the covering rate of nano-structure in the nano-structured region is measured by the scanning probe microscope (SPA-300HV, DFM) and the analytical software (See Picture 1). The covering rate of nano-structure = the surface area of the conventional support coated by nano-stucture / the total surface area of the conventional support. In the nano-structured support prepared for this example, when the concentration (w/v) of nano-particle suspension used for coating support is 1/250 or 1/175000, the covering rates of nano-structure in the nano-structured region are less than 30%, even less than 20%. However, when the concentration (w/v) of nano-particle suspension used for coating support ranges from 1/25000 to 1/50000, the covering rates of nano-structure in the nano-structured region are more than 30%, even more than 40%.

In the implementation example, the surface area increasing rate of nano-structure is obtained by measuring the specific surface areas of the support and nano-structured support respectively, and then calculating the ratio between the two. (the surface area increasing rate= (the specific surface area of nano-structured support / the specific surface area of the conventional support therein) X 100% ) , wherein the nano-structured support is prepared by using the said nanoparticle to coat the whole support via the said method. In the said nano-structured support prepared in the exmaple, when the concentration (w/v) of nano-particle suspension used for coating support is 1/250 or 1/175000, the surface area increasing rates are less than 300%, even less than 200%. However, when the concentration (w/v) of nano-particle suspension ranges from 1/25000 and 1/50000, the surface area increasing rates are all more than 200%, even more than 300%, and for some individual cases the rate is more than 500%.

In this implementation example, the adsorbability increasing rate of the nanometer region are obtained through testing the adsorbabilities of the support and nano-structured support respectively ( the adsorbability increasing rate= (the adsorbability of nano-structured area / the adsorbability of the conventional support therein) × 100%), wherein the nano-structured support is prepared by using the said nanoparticle to coat the whole support via the said method. The test is executed according to the known method, and the static adsorption lasts for 30 minutes at the room temperature. In evaluation of chip substrates, measure the adsorbability of Rhodamine-labeled anti-antibody (by IgG labeled by Molecular Probes F-6163,). In evaluation of micro-plate substrates, measure the adsorbability of enzyme-labeled anti-antibody (by Tiantan Biological Products Co., Ltd.). In evaluation of nano-particle/micrometer particle complex, measure the adsorbability of albuminis of the complex used as the functional matrix in column chromatography (by Tiantan Biological Products Co., Ltd.). In the said nano-structured support prepared in the exmaple, when the concentration (w/v) of nano-particle suspension used for coating support is 1/250 or 1/175000, the adsorbability is less than 130%, even less than 115%. However, when the concentration (w/v) of nano-particle suspension ranges from 1/25000 and 1/50000, the adsorbability is all more than 115%, even more than 130%, and for some individual cases the rate is more than 180%.

In this implementation example, the adsorption-attenuating rate is obtained through measuring respectively the adsorption decrease in the support and nano-structured support along with the decrease in adsorbates concentration (decrease of each time is measured as 10 time dilution). (The adsorption-attenuating rate=(the adsorption-attenuating velocity of the nano-structured area that decreases with the decreasing lower limit of adsorbate concentration / the adsorption- attenuating velocity of the conventional support therein that decreases with the decreasing lower limit of adsorbate concentration) × 100%) , wherein, the nano-structured support is obtained by using the said nanopsrticle to coat the whole support via the said method. The adsorption-attenuating velocity of the decreasing lower limit of adsorbate concentration = (adsorption 2 - adsorption 1) / (adsorbate concentration 2 - adsorbate concentration 1).

The adsorption amount is measured in the same way as in the adsorbability measurement. In the nano-structured supports prepared in this example, the adsorption-attenuating rate is more than 90%, even near 100%, when the supports are coated with nano-particle suspension whose concentration (w/v) is 1/250 or 1/175000. However, when the supports are coated with nano-particle suspensions whose concentration (w/v) ranges from 1/25000 to 1/50000, the adsorption-attenuating rate is less than 90%, even less than 80%, and for some individual cases the rate is less than 65%.

In this implementation example, DFM measurment is conducted at Analysis & Test Center of Chengdu Electronic Technology University. Scanning electronic microscope detection is conducted at Analysis & Test Center of Sichuan University. The detection of specific surface area is executed in Analytical laboratory, Organic Chemistry Institute of China Academy of Sciences. The adsorption of chip substrate is tested by the known chip assay methods (Refer to the following implementation examples for details) such as the establishment of the standard curve; the adsorption of the substrate of ELISA plate is tested by the known ELISA assay methods (Refer to the following implementation examples for details) such as the establishment of the standard curve; the adsorption of nanopsrticle/micrometer particles is tested by the known chromatography analysis methods (Refer to the following implementation examples for details) such as the establishment of the standard curve; Though different measuring instruments and calculation methods may bring about differences in data, still if some values are obviously bigger than others, a comparative study on them might be of significance in principle.

### Implementation example 4: the preparation of active nano-structured support (1)

The active nano-structured support prepared in this implementation example is the one used for polypeptide analysis, the nano-structured chip in particular. In the invention, the term "nano-structured chip" contains at least an active nano-structured region described in the invention (e.g. a spot in probe micro-array). A chip can possess more than one reactor and a reactor can possess more than one active-reagent-containing spots (probe spots). So long as one spot is of the active nano-structured region of the invention, the chip is considered the invented active nano-structured support or nano-structured chip. The nano-structured chips in this example include complex of active nano-particles/substrates and complex of active nano-particles / micrometer particles / substrates. The nano-structured chips in this example can be applied to other targets, such as pharmaceutics that can interact with polypeptides.

The active reagents used in this example include polypeptides (e.g. synthetic peptide EBV VCA-P18), antigens (e.g. HCV, HIV and Syphilis antigen), and antibodies (e.g. HBs antibody). The method in this example is also applicable to other active reagents, such as drugs, polysaccharides, vitamins, antibiotics, functional organisms, single-strand or multi-strand DNA, RNA, viruses, cells and their combinations.

The supports used in this example include conventional supports and nano-structured supports. The conventional supports used in this example include the chip substrates in Table 1 (slides and the derivatives of slides). The preparing method in this example is also applicable to chip substrates made of the following materials or derivatives: silica, silica gel, ceramics, metallic oxide, metal, polymer material as well as their compounds. The nano-structured support in this example is the one containing no active reagents mentioned-above, and includes the substrate of nano-structured chip prepared in Implementation Example 3.

The nano-particles used in this example are the same as those in Implementation Example 3 (nano-particles in Table 1 and nano-particle derivatives prepared in Implementation Example 1). The method in this example is also applicable to other nano-particles. The active nano-particles used in this example are those prepared in Implementation Example 2. The method in this example is also applicable to other active nano-particles. The micrometer particles used in this example are the same as those in Implementation Example 3 (micrometer particles in Table 1).

The chip prepared in this example is the one with multiple wells, and its preparing method is as follows: paint the partition structure of the chip with high-hydrophobic-silica coating material (by Chengdu Chenguang Chemical Industry Design Institute), dry it in such a way as to form a membrane whose thickness of less than 0.05mm (Refer to Chinese Patent Application: Number 03117397.7). There are 8 reaction-wells on one chip. The size of each is 4.5mm x 4.5mm, and the width of partition structure between two reaction-wells is 4.5mm. The method in this example can also be applicable to the chip with single reaction-well.
1) The preparation of active nano-particle/substrate complex (1)
   The active nano-particle/substrate complex prepared in this example is the nano-structured support of mono-active reagent, i.e. an active nano-structured region contains active nano-structured supports possessing only one type of active reagent, including substrates coated with ligand-nano-particles, ligand-nano-structure-ligand substrate, and substrates coated with ligand -nano-structure-ligand-nano-particle. The ligand mentioned in this example is used not only as reagent but also as linking agent.
   (1) Basic methods
      (a) Active-reagent immobilization method
         Spot the active reagents by the arrayer (DY-2003 biochip microarrayer) in the pattern of microarray onto the active-reagent-free nano-structured support and obtain the active nano-particle/substrate complex. The spotting method and condition used in this example are the same as those in the established methods, and numerous nano-structured chips (ligand-nano-particle-coated substrate) can be obtained this way.
      (b) Active-nano-particle immobilization method
         Make suspensions of different concentration with affinity nano-particles (See Table 5) prepared in Implementation Example 2, spot them in the pattern of microarray unto the said conventional supports (ligand-nano-particle-ligand-substrate) or nano-structured supports (substrates coated with ligand-nano-particle-ligand-nano-particle) by employing the arrayer (DY-2003 biochip arrayer), and obtain the complex of active nano-particle/substrates. In the implementation example, the reagent, such as the coupling active reagent used to fix the active nanoparticle, is sometimes immobilized onto the substrate. The spotting method and condition used in this example are the same as those in the known methods, and numerous nano-structured chips can be acquired in this method.
   (2) Chip preparation via heat treatment
      For the nano-particle immobilized on the support, heat treatment can improve its stability. For example, enclose the prepared chip with bovine serum albumin, dry it without washing away the protein and then cool it down after being heated at 50°C for 10 hours. This can improve at least the nano-structure stability of part of the chips. Many protective agents besides albumin are used in the heat treatment for active reagents, such as the sugars (dextran, maltose etc.). The heat conditions should contribute to enhancing not only the stability of nano-structure, but also that of the active reagent with the protective agent. The enhanced stability of nano-structure is evidenced by some chips (e.g. chip 19 and 20 in Table 7) that undergo heat treatment (e.g. at 37°C for over 15 hours, at 60°C over 10 hours, or at 150°C over 1 hours) in comparison with the corresponding chips without heat treatment. After being rinsed supersonically for 2 hours, the former has a higher stability than the latter. Numerous nano-structured chips with high stability can be acquired this way.
   (3) The preparation of chips or substrates with active nano-structured channels
      The nano-structured region in the invented nano-structured support can be used as transporting and separating channels for fluids. The method of preparing active nano-structured channel in this example is the same as that in Implementation Example 3. The only difference is that active nano-particle is used here to replace nano-particles (e.g. the active nanoparticles prepared in Implementation Example 2). Although the active channel in this invention is simple, professional all know that a combination of this invention with other well-known techniques will lead to an easy production of chips with various types of channels.
2) The preparation of active nano-particle/substrate complex (2)
   The active nano-particle/substrate complex prepared in the implementation example is a nano-structured support with multi-active reagents, i.e., an active nano-structured support containing two or more types of active reagents in an active nano-structured region, which includes: ligand②-nano-particle-ligand②-ligand① -nano-particle-ligand(1)-substrate and ligand② -nano-particle -ligand② - ligand① -substrate, wherein the ligand① can have the coupling reaction with ligand② and therefore bind more easily with substrates than ligand②. One type of ligand in this example (ligand②, e.g. HBsAb) is used not only as reagent, but also as linking agent; however, the other (ligand①, e.g. HBsAg) is only used as linking agent.
   In this implementation example, the preparation method of ligand②-nano-particle-ligand② - ligand① - nano-particle - ligand①- substrate is as follows: mix the suspension of ligand② -nano-particle-ligand② whose nano-particle concentration is 1/25000 (w/v) with that of ligand①-nano-particle-ligand① at the ratio of 1:1, then spot the mixture onto the substrate via the routine method, next form 3 x 2 arrays with 2 spots on each type of active nano-particles, and enclose it with bovine serum albumin solution for use. The acquired chip is marked as No.223 and the concentration ofHBsAb is 3mg/ml at spotting.
   In this implementation example, the preparation method of ligand②-nano-particle-ligand② - ligand① -substrate complex is as follows: mix the suspension of ligand ② -nano-particle-ligand ② whose nano-particle concentration is 1/25000 (w/v) at the ratio of 1:1, then spot the mixture onto the substrate via the routine method, next form 3 x 2 arrays with 2 spots on each type of active nano-particles, and enclose it with bovine serum albumin solution for use. The acquired chip is marked as No.224 and the concentration of HBsAb is 3mg/ml at spotting.
3) The evaluation of active nano-structured support
   In the implementation example, nano-structure units and their distribution, nano-convexes and their heights, the minimum size at their half height and distribution density are measured by employing the scanning probe microscope (SPA-300HV, DFM) and the electronic scanning microscope (See Picture 2). The measuring methods are the same as those in Implementation Example 3.

In the active nano-structured support prepared in this example, the nano-structure units are arranged nonorientationally (See Picture 2). When the concentration (w/v) of the prepared nano-particle suspension used to coat the support is 1/250 or 1/175000, the height is over 3nm, and the minimum size of nano-convex at its half height is 1-500nm at least in one-dimensional size, the distribution density of nano-convex is less than 10/µm², even less than 20/µm². Moreover, the covering rate of nano-structured region in the nano-structure is less than 30%, even less than 20%. However, when the concentration (w/v) of the prepared nano-particle suspension ranges from 1/25000 to 1/50000, the height is over 3nm, and the minimum size of nano-convex at its half height is 1-500nm at least in one-dimensional size, the distribution density of nano-convex is more than 10/µm², even more than 20/µm². Moreover, the covering rate of nano-structured region in the nano-structure is over 30%, over 50% or even 80%. The covering rate of nano-structure = the nano-stucture-covered surface area of the conventional support /the total surface area of the conventional support.

In this implementation example, the surface increasing rate of the active nano-structured region (the surface increasing rate = (surface area of the active nano-structured region/surface area of the conventional support therein) x 100% ) is measured in the same way as in Implementation Example 3. With fewer spots on the chip, active nano-structured region is a model obtained by coating the whole substrate (e.g. epoxy-group slides in Table 3) with active nano-particles ( (HCV Ag)-LUDOX in Table 5), wherein the conventional support refers to the whole substrate (e.g. epoxy-group slides). In preparing the active nano-structured support, when the nano-particle suspensions whose concentration (w/v) is 1/250 or 1/175000 are used to coat the support, the surface increasing rate is less than 300%, even less than 200%. However, when the suspensions of same active nano-particles whose concentration (w/v) ranges from 1/25000 to 1/50000 are used to coat the same support, the surface increasing rate is more than 200%, even more than 400%, and in some individual cases, it could be more than 650%.

In this implementation example, the adsorbability increasing rate of the active nano-structured region ( adsorbability increasing rate = (adsorbability of the nano-structured region / adsorbability of the conventional support therein) X 100%) , is measured in the same way as in Implementation Example 3, that is, taking the whole support coated with active nano-particles by the said method as the active nano-structured region. In preparing the active nano-structured support in the example, when the suspensions of active nano-particle ((HCV Ag)-LUDOX in Table 5) whose concentration (w/v) is 1/250 or 1/175000 are used to coat the substrates (e.g. epoxy-group slides in Table 3), the adsorbability increasing rate is below 130%, even below 115%. However when the suspensions of same active nano-particles whose concentration (w/v) ranges from 1/25000 to 1/50000 are used to coat the same substrates, the adsorbabiltiy increasing rate is over 115%, even over 130%, and in some individual cases, it could be more than 180%.

In the implementation example, the sensitivity increasing rate is measured by comparing the ratio of analytical sensitivities between the active nano-structured region and the non-active nano-structured support that contains the same conventional support and active reagent: the sensitivity increasing rate= (sensitivity of the active the nano-structured area / sensitivity of the non-active nano-structured support) x 100%.

In the implementation example, the model of nano-structured chips refers to those prepared by spotting the suspension of active nano-particle whose concentration ranges from 1/25000 to 1/50000 (w/v) onto the substrate (e.g. epoxy-group plate in Table 3), while possessing the features of the active nano-structured region mentioned-above. The model chip of non-nano-structured active support refers to those prepared by loading the same active reagent onto the same substrate under the same condition. In preparing the nano-structured chip in the example, when the suspensions of active nano-particle whose concentration (w/v) is 1/250 or 1/175000 are used to coat the substrate, the sensitivity-increasing rate is below 150%, even below 120%. However, when the suspensions of same active nano-particle whose concentration (w/v) ranges from 1/25000 to 1/50000 are used to coat same substrate, the sensitivity-increasing rate is over 120%, even over 150%, and in some individual cases, it could be over 600%.

In the implementation example, the attenuating rate of signal identification is measured through respectively testing the attenuating rate of signal identification of nano-structured chip and non-nano-structured chip containing the same substrate and active reagent, as the target concentration decreases. Attenuating rate of signal identification = (attenuation velocity of signal identification of the active nano-structured region that moves along with the decreasing lower limit of target concentration / the attenuation velocity of signal identification of the non-nano-structured active support that moves along with the decreasing lower limit of target concentration) × 100%, wherein the attenuation velocity of signal identification that moves with the decreasing lower limit of target concentration = (identifiable signal ② - identifiable signal ① / (target concentration ② - target concentration ①). The identifiable signals (e.g. the reading on the confocal scanner of the fluorescein-labeled targets captured by active nano-structure) can be measured via the known chip measuring method. In preparing the nano-structured chip in the example, when the nano-particle suspensions whose concentration (w/v) is 1/250 or 1/175000 are used to coat the support, the attenuating rate of signal identification is more than 70%, even near 90%. However, when the suspensions of active nano-particles whose concentration (w/v) ranges from 1/25000 to 1/50000 are used to coat the same substrate, the attenuating rate of signal identification is less than 90%, even less than 70%, and in some individual cases, it could be ess than 50%.

In the implementation example, the adsorbability-attenuating rate = (adsorption attenuation velocity of the active nano-structured region that moves with the decreasing lower limit of target concentration / adsorption attenuation velocity of the conventional support that moves with the decreasing lower limit of target concentration) × 100%) . It is measured in the same way as in implementation example 3, that is, taking the whole support coated with active nano-particles by the said method as the active nano-structured region.

In preparing the active nano-structured support in this example, when the nano-particle suspensions ((HCV Ag)-LUDOX in Table 5) whose concentration (w/v) is 1/250 or 1/175000 are used to coat substrates (e.g. epoxy-group slides in Table 3), the adsorbability-attenuating rate is more than 80%, and even near 90%. However, when the suspensions of same nano-particle whose concentration (w/v) ranges from 1/25000 to 1/50000 are used to coat the same substrates, the adsorbability-attenuating rate is less than 90%, even less than 80%, and in some individual cases, it could be less than 70%.

Though different measuring instruments and calculation methods may bring about differences in data, still if some values are obviously bigger than others, a comparative study on them might be of significance in principle.

### Implementation Example 5: preparation of active nano-structured support (2)

The active nano-structured support prepared in the implementation example is used for nucleic acid analyses, and the nano-structured chip in particular is designed for such a pourpose. It can also be applicable to other targets, such as nucleic-acid-interactive pharmaceutics.

The active reagents used in the implementation example include single-strand or multi-strand DNA, RNA, and viruses. The support and nano-particle used here are the same as those in Implementation Example 4. The preparing method in the example is the same as in that in Implementation Example 4.

The evaluation method in this example is the same as that in Implementation Example 4. The active nano-support prepared in the example produces the same result as the active nano-support prepared with the same method in Implementation Example 4. When the substrate is coated with suspensions of active nano-particle whose concentration (w/v) ranges from 1/25000 to 1/50000, the obtained results are as follows: in appearance, the nano-structure units are arranged non-orientationally; the height of the nano-convexes is over 3nm and the distribution density is more than 18/µm² at the convex half height where at least one-dimensional size is 1-500nm; In features, the covering rate of nano-structure is more than 70%; the surface-increasing rate is more than 450%; the adsorbability- increasing rate is more than 160%; the adsorbability-attenuating rate is less than 70%.

### Implementation Example 6: preparation of active nano-structured support (3)

The active nano-structured support prepared in the implementation example is the compelx of active nano-particles/micrometer particles, which can be used for preparing analysis-chips or chromatography gel.
The active reagents used in the example include polypeptides (e.g. synthetic peptide EBV VCA-P18), antigens (e.g. HCV, HIV and Syphilis antigen), and antibodies (e.g. HBs antibody). The method in the example is also applicable to other active reagents, such as pharmaceutics, polysaccharides, vitamins, antibiotics, functional organisms, single-strand or multi-strand DNA, RNA, viruses, cells and their combinations.

The micrometer particles used in the example are those listed in Table 1, including conventional chromatography gel: silica gel (particle size 40-60µm, by Chemical Institute, Chinese Academy of Sciences), and Sephadex (by Pharmacia Corp.). The preparing method in the example is also applicable to micrometer particles made of the following materials or their derivatives : ceramics, metallic oxide, metals, other polymer materials and their compounds, as well as micrometer particle coated with nano-particles.

The nano-particles used in the example are the same as those used in Implementation Example 3 (nano-particles listed in Table 1 and nano-particle derivatives prepared in Implementation Example 1). The method in the example is also applicable to other nano-particles. The active nano-particle used in the example is the one prepared in Implementation Example 2. The method in the example is also applicable to other active nano-particles. The micrometer particle used in the example is the same as that used in Implementation Example 3 (micrometer particles listed in Table 1).
1) Preparing method
   Three methods in the example are adopted to prepare the active nano-particle/micrometer particle complex: a) coat active reagents onto the nano-particle/micrometer particle complex prepared in Implementation Example 3; b) prepare the complex of active nano-particles/micrometer particles by mixing the active nano-particles prepared in Implementation Example 2 with other particles according to the method specified in Implementation Example 3 for preparing nano-particle/micrometer particle complex; c) coat the active nano-particles prepared in Implementation Example 2 onto the complex of the nano-particles/micrometer particles in Implementation Example 3 according to the method specified in Implementation Example 3 for preparing nano-particle/micrometer particle complex. In the implementation example, active reagent is coated onto the nano-particle/micrometer particle complex in accordance with the known methods, e.g. the method of coating active reagent onto micrometer particles in the preparation of affinity chromatography gel. Table 7 contains part of the active nano-particle/micrometer particle complexes prepared in the implementation example.
2) Evaluation method
   The evaluation method in this implementation example is the same as that of Implementation Example 4. The active nanometer support prepared in the example produces a result similar to that of active nanometer support prepared with same method in Implementation Example 4. For example, in this implementation, complexes ofHCV AG)-LuDOX/silica gel and (HCV AG)-LuDOX/Sephadex are obtained by coating silica gel and Sephadex A50 with HCV AG) -LuDOX respectively. When suspensions of the same active nano-particles whose concentration ranges (w/v) from 1/25000 to 1/50000 are employed in the preparation, the following results are obtained: in appearance, the nano-structure units are arranged non-orientationally; the height of the nano-convex is over 3nm and the distribution density is more than 15/µm² at the convex half height where at least one-dimensional size is1-500nm; in features, the covering rate of nano-structure is more than 50%; the surface increasing rate is more than 350%; the adsorbability-increasing rate is more than 130%; the adsorbability-attenuating rate is less than 80%.

### Implementation Example 7: preparation of nano-particle marker

The labeling complex prepared in this implementation example is made of active labeling reagent/nano-particles/molecular markers, and used as the nano-particle marker in the chip.
The labeling active reagents used in the example include polypeptides, such as HCV antigens and goat-anti-human secondary antibody (by Beijing Tiantan Biological products Co., Ltd.). The method in the example is also applicable to other active labeling reagents, e.g. pharmaceutics, polysaccharides, vitamins, antibiotics, functional organisms, single-strand or multi-strand DNA, RNA, viruses, cells and their combinations.

Not a labeling enhancer itself, the nano-particle used in the example is the non-magnetic inorganic non-metal particle with a particle diameter 1-100nm, including inorganic non-metal nano-particles in Table 1 and the derivatives of these inorganic non-metal nano-particles prepared in Implementation Example 1. The method in the example is also applicable to other non-magnetic inorganic non-metal nano-particles. The active nano-particles used in the example are those based on non-magnetic inorganic non-metal nano-particles prepared in Implementation Example 2. The micrometer particles used in the example are the same as those in Implementation Example 3 (the micrometer particles listed in Table 1). The molecular labeling material in the example is Rhodamine (by Molecular Probes Corp.). The method in the example is also applicable to other labeling materials, e.g. fluorescent material, chemiluminescent material, chemiluminescent catalyst, colored metal salt, dye and pigment.

The label control in the implementation example is a routin label not treated with nano-particle solution (Rhodamine-labeled goat anti-human secondary antibody, by Jackson ImmunoResearch Laboratories, USA)
1) Preparation
The preparing method of the compound of ligand-molecule labeling material in the implementation example is the known method of preparing the Rhodamine-labeling anti-antibody.
The preparing method of active nano-particles in this implementation example is as follows: set nano-particles in ultrasonic vibration, made them dispersively into nano-particle solutions whose concentration ranges from 1/50 (1/125) to 1/10000 (1/25000) (w/v), mix the solutions with active labeling reagent solution whose concentration is 2mg/ml at 1: 1 ratio respectively, and proceed with the reaction at room temperature away from light for 2 hours. The method of purifying the mixture is: instill the mixed resultant into a rotating tube filled with gel, centrifuge it under the condition of 4000 r/min and remove the liquid from the collecting tube for use.
The method of preparing the complex of nano-particle-molecular labeling material in this implementation example is as follows: set nano-particles in ultrasonic vibration, made them dispersively into nano-particle solutions whose concentration is 1/1000 ( 1/125) (w/v), mix the solutions with molecular labeling material solution whose concentration is 2mg/ml at 1: 1 ratio respectively, and proceed with the reaction at room temperature for 1 hour. If necessary, the method of purifying the mixture is: instill the mixed resultant into a rotating tube filled with gel, centrifuge it under the condition of 4000 r/min and remove the liquid from the collecting tube for use.
2) Preparation of the complex of ligand / nano-particle / molecule labeling mateiral
In the preparation method of this Implementation Example, the combination of the said ligand, molecular labeling material and nano-particles includes one or more of the following ways: a) let the active nano-particles combine with molecular labeling material; b) let the nanoparticle-molecular labeling material combine with ligand; c) let molecular labeling material and ligand prepared through the known method combine with nano-particles; d) combine ligand, molecular labeling material and nano-particle simultaneously, wherein the resultant can be either mixture or purified substance. Table 6 contains part of the complex of ligand / nano-particle / molecular labeling material prepared in the example.
3) Comparison
The chips employed in this implementation example are prepared via the known chip preparating method (substrates are epoxy glass slids, and ligands are hepatitis C virus antigens (HCV AG) and human immunodeficiency virus antigens (HIV AG) respectively.)
The comparative method used here is the same as that of chip application in Implementation Example 10. Table 9 contains the comparative results among part of the markers prepared in the implementation example, which indicates that its sensitivity is obviously improved in comparison with its control.

**Table 6**

| Marker | Nano-particle | Preparin g method | Test result | | | |
|---|---|---|---|---|---|---|
| | | | Dilution multiple | Sample1 | Sample2 | Sample3 |
| Marker of the control | No | | 200 | + | + | - |
| Marker of the control | No | | 400 | - | - | - |
| Marker 1 | Silica nano-particle (STN-3) | (A) | 400 | + | + | - |
| Marker 2 | Silica nano-particle (STN-3) | (B) | 400 | + | + | - |
| Marker 3 | Silica nano-particle (STN-3) | (C) | 400 | + | + | - |
| Marker 4 | Silica nano-particle | (D) | 400 | + | + | - |
| | (STN-3) | | | | | |
| Marker 5 | Polyvinylpyrrolid one-coated nano-particle | (C) | 400 | + | + | - |
| Marker 6 | Amino diazane-polylysine coated nano-particle | (C) | 400 | + | + | - |

### Implementation Example 8: preparation and application of chip marking system (1)

The chip marking system prepared in the implementation example comprises markers, colorants, and optional color-controlling agents and color stabilizers.
1) Preparation
   The chip markers prepared in the implementation example are the following respectively: ①the complex of labeling active reagent/nano-structure/calalyst, ②the complex of labeling active reagent/ nano-structure/calalyst and colorant, and ③the mixtures of either ① and the complex of labeling active reagent / nano-structure /colorant or ① and the complext of labeling active reagent /colorant.
   The labeling active reagent, nano-particle and active nano-particle used in the implementation example are the same as those in Implementation Example 7. The catalyst used in the example is the amino-group colloidal gold particle (2020) (Nanoprobes Corp.), whose diameter is 1.4nm. The method of the implementation example is also applicable to other catalysts, e.g. enzyme catalyst. The colorants used in the example include silver compound (AgNO₃) and crystal violet. The said method is also applicable to other metal compounds and dyes.
   The preparing methods of the complex of labeling active reagent/nano-structure/calalyst, the complex of labeling active reagent/nano-structure/calalyst and colorant, and the complex of labeling active reagent /nano-structure / colorant are the same as those in Implementation Example 7.
   For the details of the preparation of labeling active reagent /colorant compound in the implementation example, refer to the relevant documents (patents for pigment labeling).
   Mixtures of the said complexes need certain volume ratios to produce.
2) Application

HIV/HCV antigen chip is made by spotting HIV antigen and HCV antigen onto an epoxy-group slide by employing the known method. The positive control (the mixture of HIV antibody-positive serum and HCV antibody-positive serum) and negative control (the mixture of HIV antibody-negative serum and HCV antibody-negative serum) of different concentration (PBS buffer solution is diluent) are added respectively. After one-hour reaction at room temperature, reactors are washed, and added respectively with the various said markers (e.g. amino-group colloidal gold/silica nano-particle/anti-human secondary antibody compound, amino-group colloidal gold/silica nano-particle/crystal violet/anti-human secondary antibody compound) and markers of the control (colloidal gold-labeled goat anti-human secondary antibody). After 10-minute reaction, reactors are again( it is) washed and added with Glutaric dialdehyde whose concentration is 2.5%. Then according to the instruction manual about silver enhancer (by Sigma), the mixture of solution A and solution B is added and the reaction is triggered. Then sodium thiosulfate is used to halt the reaction. Then the chip is washed and observed by naked eyes with the following results: with all the markers, the positive control serum which is diluted 5000 times presents positive result (black spots); with the invented marker, the negative control serum which is diluted 10 times presents negative result (without black spots); while with marker of the control, under the same condition, positive result (black spots) can be observed. The results suggest that the invented marker possesses a higher specificity.

### Implementation Example 9: preparation of chip marking system (2)

The chip marking system prepared in the implementation example comprises markers, colorants, and optional color-controlling agents and color stabilizers.
1) Preparation
   The chip markers prepared in the implementation example are: ①the mixture of active reagent/ catalyst compound and active reagent/ colorant, ②the mixture of active reagent / catalyst compound and active reagent/nano-structure/colorant complex, and ③the mixture of labeling active reagent / nano-structure / catalyst complex and labeling active reagent / nano-structure /colorant complex.
   The labeling active reagent, nano-particle, active nano-particle, catalyst and colorant used in this implementation example are the same as those in Implementation Example 8.
   The preparing methods of ①the labeling active reagent / nano-structure /calalyst complex, ②labeling active reagent / nano-structure / calalyst and colorant complex, ③labeling active reagent / nano-structure /colorant complex in this implementation example are the same as those in Implementation Example 7.
   For the details of the preparation of labeling active reagent /colorant compound in the implementation example, refer to the relevant documents (patents for pigment labeling).
   The labeling active reagent/ colorant compound in this implementation example is the goat anti-human anti-antibody labeled by nanometer gold of a size of 1.4nm (by Nanoprobes Corp.). Mixtures of the said complexes need certain volume ratios to produce.
2) Application

HIV/HCV antigen chip is made by spotting HIV antigen and HCV antigen onto an epoxy-group slide by employing the known method. The positive control (the mixture of HIV antibody-positive serum and HCV antibody-positive serum) and negative control (the mixture of HIV antibody-negative serum and HCV antibody-negative serum) of different concentration (PBS buffer solution is diluent) are added respectively. After one-hour reaction at room temperature, reactors are washed, and added respectively with the various said markers (e.g. amino-group colloidal gold/silica nano-particle/anti-human secondary antibody compound, amino-group colloidal gold/silica nano-particle/crystal violet/anti-human secondary antibody compound, the mixture of Amino colloidal gold/anti-human secondary antibody compound and crystal violet/anti-human secondary antibody compound) and markers of the control (colloidal gold-labeled goat anti-human secondary antibody). After 10-minute reaction, reactors are again( it is) washed and added with Glutaric dialdehyde whose concentration is 2.5%. Then according to the instruction manual about silver enhancer (by Sigma), the mixture of solution A and solution B is added and the reaction is triggered. Then sodium thiosulfate is used to halt the reaction. Then the chip is washed and observed by naked eyes with the following results: with all the markers, the positive control serum which is diluted 5000 times presents positive result (black spots); with the invented marker, the negative control serum which is diluted 15 times presents negative result (without black spots); while with marker of the control, under the same condition, positive result (black spots) can be observed. The results suggest that the invented marker possesses a higher specificity.

### Implementation Example 10: Preparation and application of chip kit(1)

1) Preparation
   The kit in this implementation example comprises the invented nano-structured chip prepared in Implementation Example 4.
2) Application
   In this implementation example, Sample 1 is HCV antibody-positive serum, Sample 2 is HCV₁₊₂ antibody-positive human serum, and Sample 3 is negative control (negative control serum of HCV antibody and HCV₁₊₂ antibody). All samples are analyzed previously with the classical ELISA method when the serum is 20-time-diluted. The marker in this implementation example is Rhodamine-labeled goat anti-human secondary antibody (by Jackson ImmunoResearch Laboratories, Inc. USA).

In experiment, the above mentioned 3 types of samples are added respectively into the reaction-well of the chip listed in Table 10. The control chip is prepared by immobilizing the same ligand via the routine spotting method onto an epoxy group slide listed in Table 1. The loading amount is 15ul and the reaction time is 30 minutes. Then wash it for 5 times with 25ul of washing solution added each time. Then 15ul of marker is added. After the reaction, the cell (well) is washed for 5 times, with 25ul of washing solution added each time. Then it is dried and scanned with the laser power/ gain set at 35/50. The scanner used herein is a laser confocal scanner (a GMS 418 microarray scanner by Afymetrix corp.), with 532nm exciting-light wavelength and 570nm emitting-light wavelengths. The read signals are processed with software (JAGUAR II ) and negative (-) or positive (+) results can be decided by compararing the average value of the results with the Cut-off value (See Table 7). The test results indicate that the nano-structured chip of the invention presents a higher sensitivity.

**Table 7**

| Chip No. | Ligand/nano-particle/substrate complex | | Test result | | | |
|---|---|---|---|---|---|---|
| | Substrate | Ligand or active nano-particle | Sample 1 | Sample 2 | Sample 3 | Sample dilution |
| Control | Aldehyde group slide | Ligand | + | + | - | 100-fol d |
| Control | Aldehyde group slide | Ligand | - | - | - | 500-fol d |

| ligand-nano-particle-ligand-substrate complex | | | | | | |
|---|---|---|---|---|---|---|
| 201 | Aldehyde group slide | Ligand colloidal gold | + | + | ― | 500-fold |
| 202 | Aldehyde group slide | (Ligand-amino)-colloidal gold (2020) | + | + | - | 500-fold |
| 203 | Aldehyde group slide | DEAE-dextran coated nano-particle with ligand | + | + | ― | 500-fold |
| 204 | Aldehyde group slide | Polyvinylpyrrolidone-coated nano-particle with ligand | + | + | - | 500-fold |
| 205 | Aldehyde group slide | amino diazane-polylysine coated nano-particle with ligand | + | + | - | 500-fold |
| 206 | Aldehyde group slide | Ligand hydrophobic silica nano-particle (CDS7) | + | + | ― | 500-fold |
| 207 | Epoxy slide | Ligand silica nano-particle (STN-3) | + | + | ― | 500-fold |
| 208 | Epoxy slide | Ligand silica (LUDOX AS -40) | + | + | - | 500-fold |
| 209 | Epoxy slide | Ligand porous silica (AEROSIL 200) | + | + | - | 500-fold |
| 210 | Epoxy slide | Ligand titanium oxide nano-particle | + | + | - | 500-fold |
| 211 | Epoxy slide | DEAE-dextran coated nano-particle with ligand | + | + | - | 500-fold |
| 212 | Epoxy slide | Ligand Polyvinylpyrrolidone-c oated nano-particle | + | + | - | 500-fold |
| 213 | Epoxy slide | amino diazane-polylysine coated nano-particle with ligand | + | + | - | 500-fold |
| 214 | Epoxy slide | Ligand hydrophobic silica nano-particle (CDS7) | + | + | - | 500-fold |
| 215 | Amino diazane slide | Ligand hydrophobic silica nano-particle (CDS7) | + | + | - | 500-fold |
| 216 | Amino diazane slide | Ligand silica nano-particle (STN― 3) | + | + | ― | 500-fold |
| 217 | Amino diazane slide | Ligand silica (LUDOX AS-40) | + | + | - | 500fold |
| 218 | Amino diazane | amino diazane-polylysine | + | + | - | 500fold |
| | slide | coated nano-particle with ligand | | | | |
| 219 | PVP-coate d slide | Ligand hydrophobic silica nano-particle (CDS7) | + | + | ― | 500fold |
| 220 | PVP-coate d slide | Ligand silica nano-particle (STN-3) | + | + | - | 500fold |
| 221 | PVP-coate d slide | Ligand silica (LUDOX AS-40) | + | + | - | 500fold |
| 222 | PVP-coate d slide | amino diazane-polylysine coated nano-particle with ligand | + | + | - | 500fold |

| Ligand-nano-particle-ligand-nano-particle-substrate complex | | | | | | |
|---|---|---|---|---|---|---|
| 223 | Substrate 2 | Ligand silica(LUDOX AS -40) | + | + | - | 500fold |
| 224 | Substrate 2 | Amino diazane-polylysine coated nano-particle with ligand | + | + | ― | 500fold |

| | | | | | | |
|---|---|---|---|---|---|---|
| In the table, the ligands are HIV antigen and HCV antigen respectively. | | | | | | |

### Implementation Example 11: Preparation and application of chip kit (2)

1) Preparation
   The product in this implementation example is an analysis-chip kit, containing complex of ligand / nano-particle/molecular labeling material. It is the same complex prepared in Implementation Example 7. The chip in the kit is the chip control in Implementation Example 10. Thus numerous kits of different kinds can be made in accordance with this implementation example..
2) Application
   In this implementation example, the employed sampling and testing methods are the same as those in Implementation Example 10. With the invented marker, positive results can be observed from the positive serum control which is diluted 500 times; whereas with the marker of the control, only negative result can be observed under the same condition. The results therefore suggest that the invented marker presents a higher specificity.

### Implementation Example 12: Preparation and application of chip kit (3)

### 1) Preparation

The kit in this implementation example comprises the invented nano-structured chip mentioned in Implementation Example 4 and the invented marking system containing complex of ligand /nano-particle /molecular labeling material in Implementation Example 7. Thus numerous kits of different kinds can be made in accordance with this implementation example.

In this implementation example, the employed sampling and testing methods are the same as those in Implementation Example 10. The results are showed in Table 8.

**Table 8**

| Test | The composition of kit | | Sample dilution multiple | Test results | | |
|---|---|---|---|---|---|---|
| | Chip | Marker | | Sample 1 | Sample 2 | Sample 3 |
| 1 | Control | Marker of the control | 200 | + | + | + |
| 2 | Control | Marker of the control | 500 | ― | ― | + |
| 3 | Nano-structure chip | Marker of the control | 500 | + | + | + |
| 4 | Nano-structure chip | Marker of the control | 600 | - | - | + |
| 5 | Control | Marker 3 | 400 | + | + | + |
| 6 | Control | Marker 3 | 600 | - | - | + |
| 7 | Nano-structure chip | Marker 3 | 600 | + | + | + |

### Implementation Example 13: Preparation and application of chip kit (4)

### 1) Preparation

The kit in this implementation example comprises magnetic chips. The magnetic nano-particle used in this implementation example is the water-based magnetic solution (NG-21A) in Table 1; the substrate is an epoxy-group slide in Table 3; the ligand is the one employed in Implementation Example 2, namely, hepatitis B virus antigen (HCV AG) and HIV1+2 antigen.
(1) Preparation
   The magnetic nano-particle chip in this implementation example is a ligand /magnetic nano-particle /substrate complex.
   The said chip is the one with multiple reactors, wherein the preparation of multi-well-substrate is the same as that in the implementation example 4. The magnetophilic nano-particle is prepared as the active nano-particle prepared in the implementation example 2.
   Spot the prepared affinity magnetic nano-particles containing hepatitis C virus antigen (HCV AG) (1 mg/ml) and HIV1+2 antigen (1mg/ml) respectively into the wells of the multi-well substrate, form 3 x 2 arrays with 2 spots on each of the affinity magnetic nano-particles, and proceed with the reaction at 37°C for 1 hour under the effect of the external magnetic field, which help drive magnetophilic nano-particles toward the substrate and therefore become immobilized on it. Once the coating is completed, the chip is enclosed with bovine serum albumin solution for use.
   The Chip control in this implementation example is the one prepared by spotting hepatitis C virus antigen (HCV AG) (1mg/ml) and HIV₁₊₂ antigen (1mg/ml) without magnetic nano-particle onto aldehyde group plates via the known spotting method.
(2) Preparation of magnetic nano-particle markers
   The magnetic nano-particle marker in this implementation example is a complex of ligand / magnetic nano-particle / molecular labeling material, in which ligand is goat anti-human secondary antibody (by Jackson ImmunoResearch Laboratories, Inc.).
3) Preparation of magnetic chip kit
   The kit of magnetic chip in this implementation example can comprise one, two, three or four types of components. They are respectively the said magnetic nano-particle chips, magnetic nano-particle markers, magnetophilic nano-particles (enclosed with bovine serum albumin), and magnetic nano-particles (enclosed with bovine serum albumin). Different combinations of components can form different kits (See Table 9 for details).
4) Application of magnetic chip kit
   The magnetic chip kit in this implementation example is used to test the negativeness or positiveness of HIV₁₊₂ antibody and HCV antibody in a sample serum. The serum samples here are the same as those in Implementation Example 2. However, the testing method is different from the known method and the difference lies in: when the kit contains other components of magnetic particles (e.g. magnetic nano-particle) in addition to the magnetic chip, the reaction is performed at the bottom of the chip under the effect of the external magnetic field, especially the stable pulsating magnetic field. With such a magnetic effect, markers of magnetic nano-particles and the target captured by magnetophilic nano-particles are apt to move toward the bottom of the reactor. And with the effect of pulsating magnetic field, the magnetic nano-particles added to the sample are apt to join the liquid, thus helping to drive the liquid sample to flow so as to have a better mixture. Since there are a variety of kits, we will only take one kit as an example to explain its application. The application of other kits can be analogized according to this example.

The components of the kit used in this implementation example are as follows: chip control, magnetic nano-particles and magnetpophilic particles, wherein the magnetic nano-particles and magnetpophilic particles are respectively mixed with 3 types of serum samples (after the blending, the concentration of the former is 1/2000 (W/V) whereas the latter is 1/4000 (W/V) ). In the application prodecure, add 15ul of serum sample that is mixed with the nano-particles into the chip reaction-well, let it react at 37°C for 10 minutes with the effect of pulsating magnetic field, then wash it before adding 15ul of magnetic nano-marker, let it react once more for 10 minutes at 37 °C with the effect of pulsating magnetic field, wash and dry it, and then proceed with scanning and analysis through the same method described in Implementation Example 10. Results obtained are showed in Table 9.

**Table 9**

| Test | kit composition | | | | | | Sample dilution multiple | Test result | | |
|---|---|---|---|---|---|---|---|---|---|---|
| | I | II | III | IV | V | VI | | Sample 1 | Sample 2 | Sample 3 |
| 1 | √ | | | | | √ | 100 | + | + | - |
| 2 | √ | | | | | √ | 400 | - | - | - |
| 3 | | √ | | | | √ | 400 | + | + | - |
| 4 | | √ | | | | | 400 | + | + | - |
| 5 | | √ | √ | √ | | | 400 | + | + | - |
| 6 | | √ | √ | √ | √ | | 400 | + | + | - |
| 7 | √ | | √ | | | | 400 | + | - | - |
| 8 | √ | | | √ | | | 400 | + | + | - |
| 9 | √ | | | | √ | | 400 | + | + | - |

| | | | | | | | | | | |
|---|---|---|---|---|---|---|---|---|---|---|
| In the table: I ―chip control, II ―chip with magneticle nano-particle, III― magnetic nano-particle, IV―magnetophitic nano-particle, V―marker of magnetic nano-particle, VI―marker control | | | | | | | | | | |

### Implementation Example 14: Preparation and application of kit for the quantitative or/and qualitative detection of polypeptides (1)

### 1) Preparation

The kit in this implementation example is ELISA kit with active nano-structured support.

The ligand used here is synthetic peptide, EBV-VCA-P18 antigen prepared through the known method as indicated in the following document: Tranchand-Bunel, D., Auriault, C., Diesis, E., Gras-Masse, H. (1998) Detection of human antibodies using "convergent" combinatorial peptide libraries or "mixotopes" designed from a nonvariable antigen: Application to the EBV viral capsid antigen, p18, J. Peptide Res. 52, 1998, 495-508. The nano-structured ELISA plate used in this implementation example is prepared by immobilizing ligand onto the bottom of micropores in the said nano-structured microwell plate.
(1) Active nano-structured support prepared by coating the support with nano-particles:

Refer to Implementation Example 3 for the preparation and evaluation of nano-structured microwell plates. Coat 0.3 ug/ml concentration synthetic peptide onto the said nano-particle-coated microwell plates (colloidal gold-coated microwell plate, silica-coated microwell plate and silica nano-particle-coated microwell plate) and the microwall plate control (96-well polystyrene plate) respectively via the routine method of preparing ELISA plate, coat 8 wells on each of the plates and enclose them with bovine serum albumin solution for use.

The ELISA plate control in this implementation example is the ELISA plate taking the 96-welled plate as substrate and coated with the EBV-VCA-P18 antigen via the said method.

### 2) Active nano-structured support made of active nano-particles

The methods of preparing active nano-particle containing EBV-VCA-P18 antigen and silica nano-particle (STN-3) are the same as those in Implementation Example 2.

With the known ELISA plate-coating method, the active nano-particle, whose concentration of EBV VAS-P18 is 0.1ug/ml, and concentration of nano-particle is 1:25000 (w/v), is coated into 8 microwells of 96-welled microplate in Table 3. After that, the 8 microwells are enclosed by bovine serum albumin solution for use.
2) Application

The samples in this implementation example are EBV IgA positive serum and EBV IgA negative serum. All samples have been pre-assayed by the classical ELISA method under reaction condition where serum is diluted 20 times.

In experiment, add the samples into the said ELISA plate control and 3 nano-structured ELISA plates respectively, 4 reaction-wells for each sample. When loaded, the samples are diluted adequately. Add 100ul of sample each time and set the reaction temperature at 37°C. Wash them 3 times with 300ul of washing solution added each time. Add 100ul marker (the enzyme-labeled goat anti-human IgA by Beijing Tiantan Biological Products Co., Ltd) and let the reaction continue at 37°C for 30 minutes. Herein the substrate added will react under the same condition as under the classical ELISA method. Then a colorimetric analysis by the microplate reader (Thermo Labsystems, by Shanghai Lab Analytical Instrument Co., Ltd.) will be conducted to decide whether the 8 wells present negative result (-) or positive result (+) based on the Cut-off value, as showed in Table 10.

**Table 10**

| ELISA plate | ELISA plate control | ELISA plate 1 | ELISA plate 2 | ELISA plate 3 |
|---|---|---|---|---|
| Substrate | 96-welled microplate | Colloidal gold-coated microplate | Silica-coated microplate | Silica nano-particle-coate d microplate |
| Coated nano-particl e | No | Colloidal gold | Silica (LUDOX AS-40) | Silica nano-particle (STN-3) |

| Test result with 20-fold-dilution (reaction time: 30 minutes) | | | | |
|---|---|---|---|---|
| Sample 1 | + | + | + | + |
| Sample 2 | - | - | - | - |

| Test result with 50-fold-dilution (reaction time: 30 minutes) | | | | |
|---|---|---|---|---|
| Sample 1 | - | + | - | + |
| Sample 2 | - | - | - | - |

| | | | | |
|---|---|---|---|---|
| As indicated in Table 10, the nano-structured ELISA plate is more sensitive and time-saving than the ELISA plate control. | | | | |

### Implementation Example 15: Preparation and application of quantitative or/and qualitative detection of polypeptides (2)

The kit in this implementation example is a rapid test kit containing active nano-structured supports.
1) Preparation
(1) Preparation of the nano-particle-coated planar chromatography strips
   The substrates used in this implementation example are nitrocellulose strips (by Fujiang Quanzhou ChanglLi Biochemical Co., Ltd) and nylon cellulose strips (by Fujiang Quanzhou Changli Biochemical Co., Ltd). The nano-particles are silica nano-particles (STN-3) (by Zhejiang Zhoushen Mingri Nanomaterials Co., Ltd.) and silica (LUDOX AS-40) (Sigma-Aldrich Corp.)
   The preparation of nano-particle-coated planar chromatography strips in this implementation example is as follows: make nano-particle solution whose concentration ranges from 1/12500 to 1/25000 (w/v) contact with substrates, continuelet reaction at room temperature for 5 hours and then wash it repeatedly with distilled water.
   The Substrate control is nitrocellulose filmstrips and nyloncellulose film strips which are not treated by the nano-particle buffer solution.
(2) Preparation of rapid test strips of active nano-structured supports
   The ligand used in this implementation example is hepatitis C virus antigen (HCV AG) (by Hepatopathy Institute, People's Hospital of Beijing).
   In preparation, spot 0.5-mg/ml-concentration Hepatitis C virus antigen (HCV AG) onto the 4 types of nano-structured planar chromatography strips and 2 types of substrate controls (detection line) respectively, load rabbit anti-goat IgG respectively to form the quality control line via the routine method, enclose them with bovine serum albumin solution, and then add the sampling region, marking region with colloidal gold-labeled goat anti-human antibody, and water-absorbing region to form an assemblage.
   The rapid test strip control is that prepared from the substrate control therein.

2) Application
In this implementation example, Sample 1 and Sample 2 are HCV antibody positive serum and HCV antibody negative serum respectively. All samples have been checked by the classical ELISA method to assure their positiveness or negativeness.
In experiment, add the two samples into the 6 types of the said rapid test strips respectively (the sample must be diluted adequately and the loading amount is 50ul), and then add washing solution immediately till the quality control line appears on the test strip. The results are shown in Table 11.

**Table 11**

| Rapid test strip | Nano-structured planar chromatography strip | | Test | | |
|---|---|---|---|---|---|
| | Conventional substrate | Nano-particle coated | Sample 1 | Sample 2 | Test time needed |
| Test strip control | Nitrocellucose film strip | No | + | ― | 2 minutes |
| Rapid test strip coated with nano-particle | Nitrocellucose film strip | Silica (LUDOX AS-40) | + | ― | 1 minute |
| Rapid test strip coated with nano-particle | Nitrocellucose film strip | Silica nano-particle (STN-3) | + | ― | 1 minute |
| Test strip control | Nyloncellucose film strip | No | + | ― | 2 minutes |
| Rapid test strip coated with nano-particle | Nyloncellucose film strip | Silica (LUDOX AS-40) | + | ― | 1 minute |
| Rapid test strip coated with nano-particle | Nyloncellucose film strip | Silica nano-particle (STN-3) | + | ― | 1 minute |

| | | | | | |
|---|---|---|---|---|---|
| As indicated by the results in Table 11, the rapid test strip coated with nano-particle takes only half of the time needed by test strip control. | | | | | |

### Implementation Example 16: Ppreparation and application of active nano-structured support as separating media

The separating media prepared in this implementation example is a functional matrix for chromatography.

### 1) Preparation

The ligand used in this implementation example is DEAE-dextran (by Pharmacia Corp.); the nano-particle used herein is silica nano-particles (STN-3) listed in Table 1; the support used herein is silica gel particle for chromatography with average diameter 60µm (by Chemical Institute, Chinese Academy of Sciences).

In preparation, mix DEAE-dextran solution whose concentration is 1/1000000 (w/v) with silica nano-particle (STN-3) whose concentration is 1/6250 (w/v) , stir the solution for 4 hours at room temperature, then add the pre-dried silica gel particles into it, then proceed with the dextran cross-link according to the method published by one of co-inventors of this invention (Refer to the article "Coated silica supports for high-performance affinity chtomatography of proteins", Journal of Chromatography, 476 (1989) 195-203) , and obtain the complex of DEAE-dextran /nano-particle / silica gel particle. Actually, due to the introduction of nanometer dextran, a variety of functional matrixes for chromatography can be made based on the classical dextran derivation.

There are other preparing methods for DEAE-dextran / silica gel particle complex as contrast, and for details refer to the method by one of the co-inventors of this invention mentioned above.

### 1) Application

This implementation example has measured the dynamic adsorptive capacity of the said silica gel particles, DEAE-dextran / silica gel particle complex and DEAE-dextran /nano-particle / silica gel particle complex. In the measurement, the column used for filling in the said media is 0.5-cm in inner diameter and 2-cm in length and the buffer solution is 0.01 M Tris-HCL / pH 7.40 with 1 ml/min flowing velocity. The chromatograph used is HP 1090; the sample is human albumin. The measured dynamic adsorptive capacities of human albumin for silica gel particles, DEAE-dextran /silica gel particle complex and DEAE-dextran / nano-particle / silica gel particle complex are respectively 1.2 mg/ml, 7.8 mg/ml and 13.5 mg/ml, wherein the DEAE-dextran / nano-particle / silica gel particle complex presents the highest dynamic adsorptive capacity.

### Implementation Example 17: Detection of HIV nucleic acid analysis-chip

1. HIV chip preparation
The chip used in the implementation example is the invented nano-structured chip, wherein the substrate is the epoxy-group slide (See Table 3); the active reagents are the 8 unclassified target genes in HIV virus conservative regions (6 in Region SK, 1 in Region P, 1 in Region C); the active nano-particle is coated silica with active reagents (LUDOX AS-40). The active nano-particle is prepared in the same way as in Implementation Example 2, while the preparation and evaluation of nano-structured chips are the same as in Implementation Example 4.
2. Sample preparation
Extract DNA via the conventional method from blood sample that has been inactivated by boiling for 10 minutes, heat the extracted DNA up to 98°C, cool it instantly down to 4°C or below, and then treat it with denaturing buffer solution. Then remove the denaturing buffer solution, add labeling buffer solution, label the treated DNA with Rhodamine, then remove the free Rhodamine when the labeling reaction is completed, from the DNA through centrifugal chramatography and make it ready for use.
3. Detection
Mix the DNA sample derived from the above-mentioned 2 blood samples with hybrid solution (the ratios are respectively 1:9; 1:99; 1:199), load the 6 diluted samples whose concentration are different unto 6 chips (4 reaction-wells for each sample), put them into a moist case for reaction (the reaction time is 60 minutes and the temperature is 42°C), then take the chips out and wash them with buffer solution 3 times, then wash again with alcohol and put aside for drying. Scan them with a laser confocal scanner and obtain the final results from the image processing software. Table 15 contains the overall evaluations of 4 reaction-wells in a chip with multiple spots.

**Table 15**

| Test | Chip | Times of Sample dilution | Test result |
|---|---|---|---|
| 1 | Chip control | 10 | + |
| 2 | Chip control | 100 | - |
| 3 | Chip control | 400 | - |
| 4 | Nano-structured chip | 10 | + |
| 5 | Nano-structured chip | 100 | + |
| 6 | Nano-structured chip | 400 | - |

## Claims

1. A nano-structured support used for separation or/and analysis, which comprises a nano-structured region, wherein the said nano-structured region comprises a nano-structure fixed on a conventional support, wherein the said nano-structure comprises nonorientationally arrayed nano-structured units, wherein the said nano-structured units include nano-convexs **characterized by** a height over 3nm, a size 1-500nm, optimally 1-100nm at the half-height in one dimension at least, wherein the distribution density of the nano-convexs in the nano-structured region is more than 1/µm², optimally more than 10 /µm².

2. The nano-structured support according to claim 1, wherein the covering rate of the said nano-structured region is more than 20%, optimally more than 30%.

3. The nano-structured support according to claim 1 or 2, wherein the surface-increasing rate of the said nano-structured region is more than 200%, preferably more than 300%, optimally more than 400%.

4. The nano-structured support according to any of the claims from 1 to 3, wherein the adsorbability-increasing rate of the said nano-structured region is more than 115%, preferably more than 130%, and optimally more than 150%.

5. The nano-structured support according to any claim of 1 to 4, wherein the adsorbability-attenuating rate of the said nano-structured region is less than 90%, optimally less than 80%.

6. The nano-structured support according to any of the claims from 1 to 5, wherein the said nano-structure units are composed of nano-particles with a diameter of 1-500nm, preferably 1-100nm, and optimally 1-50nm.

7. The nano-structured support according to claim 6, wherein the said nano-particles include inorganic or/and organic nano-particles as well as their derivatives, wherein the said inorganic nano-particles include magnetic and/or non-magnetic inorganic nano-particles, wherein the said non-magnetic inorganic nano-particles include non-magnetic metal and/or non-metal nano-particles, wherein the said derivatives include nano-particles bound surficcially to chemical groups or/and those coated with organic compounds.

8. The nano-structured support according to claim 7, wherein the non-magnetic non-metal inorganic nano-particles include oxide nano-particles such as silica oxide particles, titanium oxide particles, or/and alumina oxide particles.

9. The nano-structured support according to claim 7, wherein the non-magnetic metal nano-particles include gold particles, vanadium particles, or/and lead particles.

10. The nano-structured support according to any of the claims from 1 to 9, wherein the conventional support includes supports made of a single group or multiple groups of the following materials or their derivatives whose sizes are over 1000nm: glass, silica, silica gel, ceramics, metallic oxide, metal, polymer material and their compounds, wherein the derivativies include those bound surficially to chemical groups or/and those coated with organic compounds.

11. The nano-structured support according to any of the claims from 1 to 9, wherein the said chemical groups include one or more of the following groups: amino-groups, aldehyde-groups, epoxy-groups, amino diazane, diethylaminoethyl, diethyl-(2-hydroxypropyl) aminoethyl, carboxymethyl, sulfopropyl, mercaptoethylpyridine, siloxanyl, thioalcohol- and alkyl-group; wherein the said coated organic compounds include one or more of following substances: surfactants such as polyvinylpyrrolidone and Tween, polyelectrolyte such as polyamino acids, oleophilic compounds such as polysiloxane, ion exchange polymers that include dextran derivatives, agarose derivatives, cellulose derivatives, polyacrylamide; and affinants such as heparin natrium, antigen and antibody.

12. The nano-structured support according to any of the claims from 1 to 11, wherein the nano-structured support includes nano-particles/substrate complex, nano-particles/micro-particle complex; or nano-particles/micro-particles/ substrate complex.

13. The nano-structured support mentioned in any of the claims from 1 to 12, which presents itself in one of the following forms. 1). nano-structured substrate for analysis-chip; 2) nano-structured microplate for Elisa plate; 3) nano-structured substrate for planar chromatography; 4) nano-structured matrix for separation; 5).matrix for separation comprising nano-structured chromatography gel.

14. A method of preparing the said nano-structured support mentioned in any of the claims from 1 to 13, which comprises the contact between the suspension of the nano-particles and the conventional support as well as the reaction of immobilization, wherein the nano-particles in the suspension have a weight/volume concentration of 1/500 to 1/150000 g/ml, or a mole concentration of 0.12-37.4nM.

15. The method according to claim 14, wherein the weight/volume concentration is 1/5000 to 1/150000 g/ml, or the mole concentration is 0.12-3.74nM .

16. The method according to claim 14, wherein the weight/volume concentration is 1/20000 to 1/60000 g/ml, or the mole concentration is 0.31 to 0.93nM.

17. The method mentioned in any of the claims from 14 to 16, which comprises also one or more of the following functionalizational treatments:
1) Functionalizing the nano-particles or/and the conventional support first, and then conducting the contact and immobilization;
2) Conducting the contact and immobilization first, and then functionalizing the nano-particles or/and the conventional support;
3) functionalizing the nano-particles or/and the conventional support first, conducting the contact and immobilization next, and then re-functionalizing the nano-particles or/and the conventional support ;
wherein the said functionalization includes introduction of the said chemical groups on the surface or/and the coated organic compounds.

18. The method mentioned in any of the claims from 14 to 17, which comprises also a chemical cross-linking tretment after the immobilization.

19. The method mentioned in any of the claims from 14 to 17, which comprises also a heat treatment after the immobilization, wherein the heat treatment is processed at over 30°C, preferably over 37°C, optimally over 40°C, but the heating begins under the sintering point of the said support and cooling takes place subsequently.

20. An active nano-structured support used for separation or/and analysis reactors, which comprises an active nano-structured region, wherein the active nano-structured region comprises active nano-structure immobilized on a conventional support, wherein the active nano-structure comprises nonorientationally arrayed nano-structured units and active reagents bound thereon, wherein the nano-structured units include nano-convexs **characterized by** a height of more than 3nm, a size of 1-500nm, optimally 1-100nm at the half-height in one dimension at least, wherein the distribution density of the nano-convexs in the nano-structured region is more than 1/µm² , optimally more than 10 /µm².

21. The active nano-structured support according to claim 20, wherein the covering rate of the said active nano-structured region is more than 30%, optimally more than 75%.

22. The active nano-structured support according to claim 20 or 21, wherein the surface-increasing rate of the active nano-structured region is more than 200%, preferably more than 400%, optimally more than 600%; or/and the adsorbability-increasing rate is more than 115%, preferably more than 130%, and optimally more than 150%.

23. The active nano-structured support according to either claim 20, 21 or 22, wherein the sensibility-increasing rate of the active nano-structured region is more than 120%, preferably more than 150%, optimally more than 200%.

24. The active nano-structured support according to any of the claims from 20 to 23,
wherein the attenuating rate of signal identification of the said active nano-structured region is less than 90%, optimally less than 70%, or/and the adsorption-attenuating rate is less than 90%, optimally less than 80%.

25. The active nano-structured support according to any of the claims from 20 to 24,
wherein the target of the said separation or/and analysis includes polypeptide or/and other drugs that interact with polypeptide.

26. The active nano-structured support according to any of the claims from 20 to 24,
wherein the target of the said separation or/and analysis includes nucleic acid or/and other drugs that interact with nucleic acid.

27. The active nano-structured support mentioned in any of the claims from 20 to claim 26, which is a complex of active nano-particles and the support, wherein the active nano-particles comprise nano-particles and one kind or more of active reagents immobilized thereon or any linking agent, wherein the nano-particles have a diameter of 1-500 nm, preferably 1-100nm, and optimally 1-50nm, wherein the said support is a conventional support or the nano-structured support mentioned in any of the claims from 1 to 14.

28. The active nano-structured support according to claim 27, wherein the said nano-particles include those described in any of the claims from 6 to 11, optimally the non-magnetic non-metal inorganic nano-particles and/or their derivatives.

29. The active nano-structured support mentioned in claim 27 or 28, which includes one of the following combinations: 1). active nano-particles / substrate complexes; 2). active ano-particles / micro-particle complexes; 3).active nano-particles / nano-structured substrate complexes; 4).active nano-particles / nano-structured micro-particle complexes; and 5).any combination from the above mentioned complexes.

30. The active nano-structured support mentioned in any of the claims from 20 to 26, which is a complex of the active agents and the nano-structured support described in any of the claims from 1 to 13.

31. The active nano-structured support mentioned in claim 30, which includes one of the following combinations: 1). active reagents/ nano-structured substrate complexes; 2).active reagents/ nano-structured micro-particle complexes ; and 3). any combination from the above mentioned complexes.

32. The active nano-structured support mentioned in any of the claims from 20 to 31, which includes nano-structured analysis-chips.

33. The active nano-structured support mentioned in any of the claims from 20 to 31, which includes one of the following groups: 1). nana-structured ELSA plates; 2). nano-structured planar-chromatography strips; and 3). active nano-structured matrix for separation, including active nano-structured chromatography gel.

34. A method of preparing the said active nano-structured support mentioned in any of the claims from 21 to 33, which comprises the contact between the suspension of the nano-particles and the conventional support as well as the reaction of immobilization,
wherein the nano-particles in the suspension have a weight/volume concentration of 1/500 to 1/150000 g/ml, or a mole concentration of 0.12-37.4nM, wherein the said contact refers to point-contact whose diameter is less than 0.5mm, or surface-contact whose diameter is more than 0.5mm.

35. The method according to claim 34, wherein the weight/volume concentration of nano-particles ranges from 1/500 to 1/150000(g/ml), or ranges from 0.12 to 37.4n for mole concentration of nano-particles.

36. The method according to claim 34, wherein the weight/volume concentration of nano-particles ranges from from 1/20000 to 1/60000 (g/ml); or ranges from 0.31 to 0.93nM for mole concentration of nano-particles.

37. The method mentioned in any of the claims from 34 to 36, which comprises also a chemical cross-linking after the immobilization.

38. The method mentioned in any of the claims from 34 to 37, which comprises also a heat treatment after the reaction of immobilization, wherein the heat treatment includes the heating at over 30°C, preferably over 37°C, optimally over 40°C, but below the sintering point of the said support, with or without the protection of active reagent protectant as well as the subsequent cooling.

39. An analytical or/and separating device, which comprises the active nano-structured support mentioned in any of the claims from 21 to 33, or/and the nano-structured support mentioned in any of the claims from 1 to 14.

40. The device mentioned in claim 39, which comprises one of the following combinations of analysis-chips: 1). analysis-chips with microarray; 2). analysis-chips with microarray and channels; 3). analysis-chips with channels, wherein the said channels include nano-structured channels with nano-structured supports, or/and active nano-structured channels with active nano-structured supports.

41. The device accoding to claim 39, which includes one of the following devices: 1).ELSA plate; 2). planar-chromatography strip; and 3).device comprising separation matrix, such as chromatography column.

42. A marking system for quantitative or/and qualitative analysis, which comprises at least one marker which is a complex of marking active reagents, nano-structures, and molecular labeling reagents, wherein the said nano-structure includes nano-particles or/and structures consisting of nano-particles, wherein the nano-particles are non-magnetic non-metal inorganic particles with a diameter of 1-100nm, and moreover, they are not reinforcing agents for labeling.

43. The marking system according to claim 42, wherein the non-magnetic non-metal inorganic particles include the non-magnetic non-metal inorganic particle described in claim 8, 9 or 10.

44. The marking system according to claim 42 or 43, wherein the molecular labeling reagents include one or more of the following materials: fluorescent material, chemiluminescent material, chemiluminescent catalysts, non-ferrous metal salts, dyes and pigments.

45. A method of quantitative or/and qualitative analysis, which comprises the analytical or/and separating device mentioned in any of the claims from 39 to 41 to capture, deliver, or/and separate sample targets as well as comprising the marking system mentioned in any of the claims from 42 to 44 for labeling.

46. A kit for quantitative or/and qualitative analysis, which comprises the analytical or/and separating device mentioned in any of the claims from 39 to 41 as well as comprising the marking system mentioned in any of the claims from 42 to 44 for labeling.

47. The kit mentioned in claim 46, which is a kit used for polypeptides or/and polypeptide-related drug analyses, including 1) analysis-chip kits, 2) ELISA kits or 3) planar-chromatography kits.

48. The kit mentioned in claim 46, which is a kit used for nucleic acid or/and nucleic acid-related drug analyses.

49. A method of quantitative or/and qualitative analyses, which comprises the analytical or/and separating device mentioned in any of the claims from 39 to 41 to capture, deliver or/and separate sample targets.

50. A kit for quantitative or/and qualitative analyses, which comprises the analytical or/and separating device mentioned in any of the claims from 39 to 41.

51. The kit mentioned in claim 50, which is a kit used for polypeptides or/and polypeptide-related drug analyses, including 1) analysis-chip kits, 2) ELISA kits or 3) planar-chromatography kits.

52. The kit mentioned in claim 50, which is a kit used for nucleic acid or/and nucleic acid-related drug analyses.

53. A method of quantitative or/and qualitative analyses, which comprises the marking system for quantitative or/and qualitative analyses mentioned in any of the claims from 42 to 44.

54. A kit for quantitative or/and qualitative analyses, which comprises the marking system for quantitative or/and qualitative analyses mentioned in any of the claims from 42 to 44.

55. The kit mentioned in claim 54, which is a kit used for polypeptides or/and polypeptide-related drug analyses, including 1) analysis-chip kits, 2) ELISA kits or 3) planar-chromatography kits.

56. The kit mentioned in claim 54, which is a kit for nucleic acid or/and nucleic acid-related drug analyses.

57. An analysis-chip detecting method, which comprises the following procedures:
1). Preparation for samples, analysis-chips and marking systems, wherein the samples may contain targets; wherein the analysis-chips comprise arrays of immobilizing active reagents; wherein the marking system comprises markers, stain, and optional color-control agents and color stabilizers; wherein the marker comprises a complex of active reagents for marking, nano-structures, and catalysts; wherein the nano-structures include nano-particles or/and structures composed of nano-particles; wherein the nano-particles are selected from inorganic particles, organic particles, or/and derivatives thereof with a diameter of 1-100nm; wherein the inorganic nano-particles include magnetic inorganic nano-particles and/or non-magnetic inorganic nano-particles; wherein the non-magnetic inorganic nano-particles include non-magnetic metal nano-particles and/or non-magnetic non-metal nano-particles; and wherein the derivatives include nano-particles bound with the surface of chemical groups or/and nano-particles coated with organic compounds.
2) The said target is fixed by the said immobilizing active reagent, and then the said nano-structure in the marker and the catalyst thereon are fixed via the reaction between the target and the active marking reagent in the marker; or
3) The said target is made fixed on the markers partially or completely, and then the nano-structure in the said marker and the catalyst thereon are fixed via the reaction between the target and the immobilizing active reagent;
4) Add the said stain and proceed with, upon the impact of the said catalyst, the coloration on the said nano-structure rather than on the connecting site between the immobilizing active reagent and the target, wherein the coloration includes the use of optional color-control agent and color stabilizer;
5) Proceed with detection and analyses of the results of the coloration reaction described in procedure 4) as well as the results of the coloration described in procedure 2) or 3).

58. The method according to claim 57, wherein the marker also comprises stain, with which staining is performed before, during or/and after the coloration reaction, wherein the said stain presents in one or more of the following markers: 1) active labeling reagent/nano-structure/catalyst/ stain complex; 2) active labeling reagent/ nano-structure /stain complex; and 3) active labeling reagent/colorant compound, wherein the nano-structure refers to that mentioned in Claim 57.

59. The method according to claim 57 or 58, wherein the said nano-particles in the marking system include the nano-particles described in any of the claims from 6 to 11.

60. The method according to claim 57 or 58, wherein the catalyst includes metal catalyst or/and enzyme catalyst in reduction reaction of metal compounds; wherein the coloring agent refers to dyes and the colorant includes metal compounds.

61. An analysis-chip detecting method, which comprises the following procedures:
1). Preparation for samples, analysis-chips and marking systems, wherein the samples may contain targets; wherein the analysis-chips comprise arrays of immobilizing active reagents;
wherein the marking system comprises markers, stain, and optional color-control agents and color stabilizers; wherein the marker comprises active reagents, catalysts, stain and optional nano-structures, wherein the nano-structures, stain, catalysts and coloring agents refer respectively to those described in any of the claims from 57 to 60;
2) Add the said sample to the analysis-chip, and after the reaction between the immobilizing active reagents and the target which may exist, add the marker and coloring agent and stain and color the site of reaction; or
3) Mix the said sample with the marker partially or completely, add the mixture to the analysis-chip after the reaction between the active marking reagent and the target which may exist, and then add the coloring agent (to the said analysis-chip) and stain and color the site of the reaction after the reaction between the immobilizing active reagent and the target which may exist;
4) Proceed with detection and analyses of the results of the staining and coloration described in procedure 2) or 3).

62. A chip kit, comprising making systems, wherein at least one of them is the said marking system claimed by any of the claims from 57 to claim 60, or by claim 61.

63. The kit mentioned in claim 62, which is used for analyzing polypeptides or/and polypeptide-related drugs.

64. The kit mentioned in claim 62, which is used for analyzing nucleic acid or/and nucleic acid-related drugs.

65. A testing method of polypeptide analysis-chip, comprising one or more of the following procedures:
1) Mix samples with magnetic nano-particles;
2) Mix samples with active reagents/magnetic nano-particle complexes;
3) Make samples contact and react with nano-structured chips either with or without the external magnetic field, wherein the said nano-structures refer to nano-particles;
4). Use complexes of active reagents, magnetic nano-particles, molecular labeling material for marking, either with or without the external magnetic field, wherein the said complex is a mixture comprising one or more molecular labeling reagents, magnetic nano-particles, active reagents and optional sealing mixture or purified material.
Wherein the said magnetic nano-particles have at least one dimensional size as 1-200nm, preferably 1-100nm, optimally 1-50nm, and furthermore the particles themselves are not magnetic particles or derivatives that serve as molecular marker enhancer;
The said active reagents are selected from the following materials which can interact with polypeptides: polypeptides, polysaccharides, vitamins, antibiotics, viruses, cells and functional organisms; the said complex of active reagents, magnetic nano-particles and molecular labeling material presents, in the process of marking , a magnetic nano-particle concentration of more than 1/30000(g/ml) or more than 0.25nM in nano-particle mole concentration, preferably more than 1/3000(g/ml) or more than 2.4nM in nano-particle mole concentration, optimally more than 1/500(g/ml) or more than 15.0nM in nano-particle mole concentration.

66. The method according to claim 65, wherein the said magnetic nano-particles are selected from ferrite and its derivatives (including ferroso-ferric oxide and iron sesquioxide);
wherein the derivatives include those whose surfaces are modified by chemical groups, or/and those coated with functional organics.

67. The method according to claim 65 or claim 66, wherein the said external magnetic field is in pulsed wave mode.

68. A analysis-chip kit for polypeptide test, which comprises the magnetic nano-particles described in claim 65 or claim 66.

69. A separation method, which comprises the separating device mentioned in any of the claims from 39 to 41.
